# EUROPEAN PATENT APPLICATION

(11) **EP 1 607 392 A1**
(43) Date of publication of application: **21.12.2005**
(21) Application number: 04720715.4
(22) Date of filing: 15.03.2004
(51) Int. Cl.: C07D 271/10, C09K 11/06, C07C 13/567, C07C 1/34

(54) **BLUE LIGHT-EMITTING COMPOUND, METHOD FOR PRODUCING SAME AND LIGHT-EMITTING DEVICE UTILIZING SAME**

(30) Priority: 17.03.2003 JP 2003072773; 16.05.2003 JP 2003139675
(71) Applicant: HIROSE ENGINEERING CO., LTD., Tokyo 146-0092 (JP)
(72) Inventor: NAKAYA, Tadao c/o Hirose Engineering Co.,Ltd., Tokyo 146-0092 (JP); MATSUMOTO, Ryoji c/o Hirose Engineering Co.,Ltd., Tokyo 146-0092 (JP); TOBITA, Michiaki c/o Hirose Engineering Co.,Ltd, Tokyo 146-0092 (JP); SAIKAWA, Tomoyuki c/o Hirose Engineering Co.,Ltd, Tokyo 146-0092 (JP); ETO, Naonobu c/o Hirose Engineering Co.,Ltd, Tokyo 146-0092 (JP); ISHITOBI, Tatsuro c/o Hirose Engineering Co.,Ltd, Tokyo 146-0092 (JP)
(74) Representative: Olgemöller, Luitgard Maria
(86) International application number: PCT/JP2004/003418
(87) International publication number: WO 2004/083194

(57) **Abstract**

The present invention provides blue-light emitting compounds capable of emitting blue light at a high luminance for a long time upon the application of electric energy, processes of producing the compounds, and luminescent elements including the blue light-emitting compounds. One of the compounds according to the present invention is characterized by the chemical structure represented by formula (1).

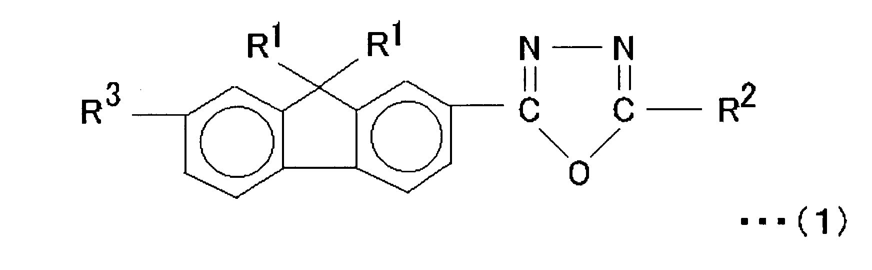

## Description

### Technical Field

The present invention relates to blue light-emitting compounds, processes of preparing the blue light-emitting compounds, and luminescent elements including the compounds. More specifically, this invention relates to blue-light emitting compounds capable of emitting blue light at a high luminance for a long time upon the application of electric energy, processes of producing the compounds, and luminescent elements including the blue light-emitting compounds.

### Background Art

For organic electroluminescent elements, which are often abbreviated to "organic EL elements", have been proposed various luminescent compounds. However, luminescent compounds that are capable of emitting blue light for a long time and excellent in durability have not been developed.

The objective of the present invention is to provide blue light-emitting compounds capable of emitting blue light at a high luminance for a long time, processes for producing them, and luminescent elements including them.

### Summary of the Invention

In order to achieve the objective, the present invention provides a blue light-emitting compound having a chemical structure represented by formula (1): 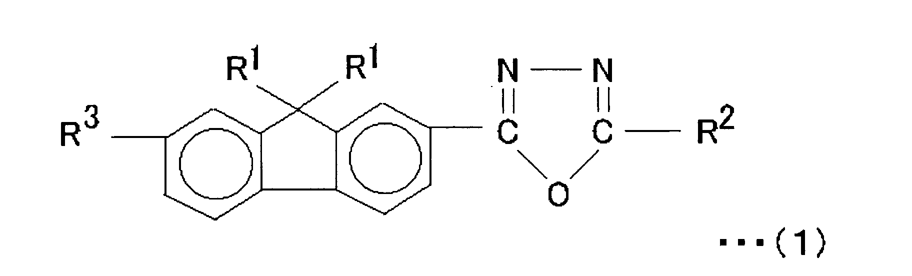 wherein R¹ is a hydrogen atom, an alkyl group having 1 to 15 carbon atoms, a cycloalkyl group having 6 to 15 carbon atoms, or an aryl group represented by one of formulas (1-1) to (1-4), wherein two R¹s may be the same or different from each other; R² is an aryl group represented one of formulas (1-1) to (1-4), or furyl group; and R³ is a group represented by formula (2) or a hydrogen atom.

Formula (1-1) is: 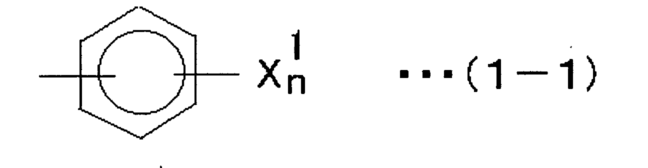 wherein X¹ is an alkyl group having 1 to 10 carbon atoms, an alkyl group having 1 to 10 carbon atoms, at least one hydrogen atom of which is replaced with a fluorine atom, or a hydrogen atom, and n denotes an integer of 1 to 5.

Formula (1-2) is: 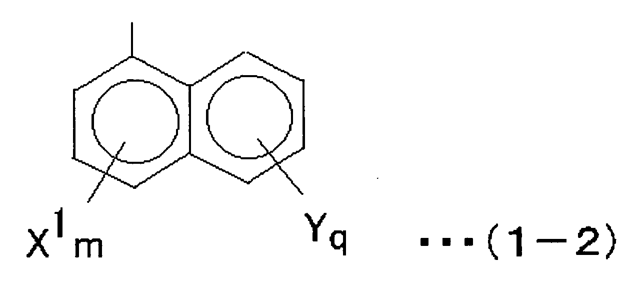 wherein X¹ means the same as the above; Y means an alkyl group having 1 to 10 carbon atoms, an alkyl group having 1 to 10 carbon atoms, at least one hydrogen atom of which is replaced with a fluorine atom, or a hydrogen atom; m denotes an integer from 1 to 3; q denotes an integer from 1 to 4; and X¹ and Y may be the same or different from each other.

Formula (1-3) is: 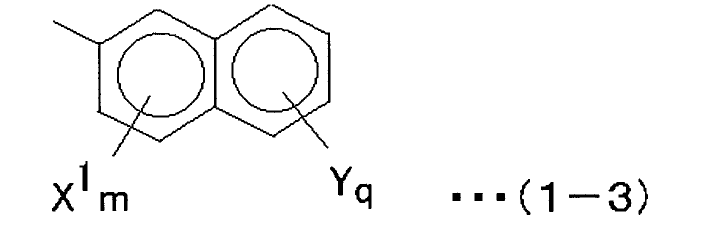 wherein X¹, Y, m and q denote the same as the above defined, and X¹ and Y may be the same or different from each other.

Formula (1-4) is: 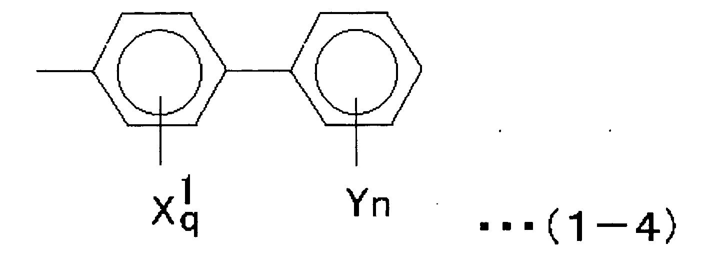 wherein X¹, Y, n, and q denote the same as those defined above, and X¹ and Y may be the same or different from each other.

Formula (2) is: 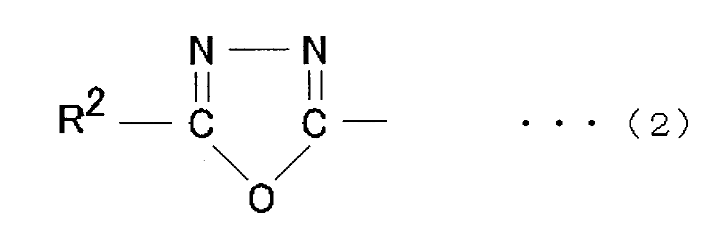 wherein R² denotes the same as that defined above; and when R³ in formula (1) is the group represented by formula (2), R² bonded to the oxadiazolyl group in formula (2) may be the same as, or different from R² bonded to the oxadiazolyl group in formula (1).

The present invention also provides a blue light-emitting compound having a chemical structure represented by formula (3) : 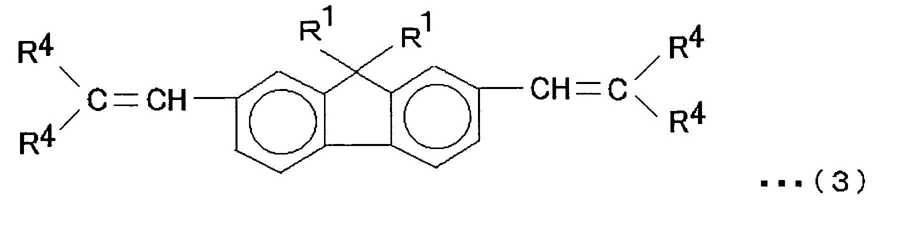 wherein R¹ denotes the same as that defined in the compound represented in formula (1); and R⁴ denotes a hydrogen atom, or an aryl group represented by formula (3-1) or (3-2) wherein four R⁴s may be the same or different from each other.

Formula (3-1) is: 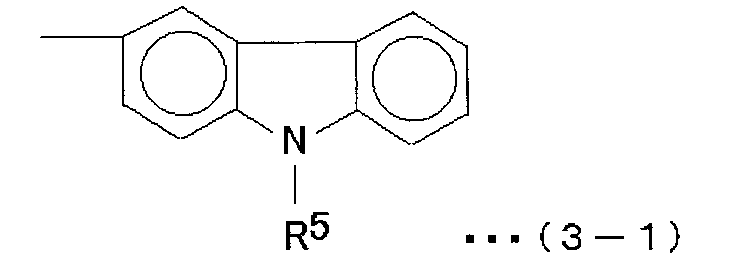 wherein R⁵ denotes a hydrogen atom or an alkyl group with 1 to 5 carbon atoms.

Formula (3-2) is: 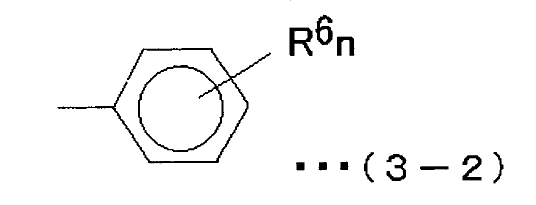 wherein R⁶ denotes a hydrogen atom or an alkyl group with 1 to 5 carbon atoms, and n denotes the same as that defined in the group represented by formula (1-1).

The present invention further provides a process for producing a blue light-emitting compound represented by formula (6), which is an embodiment of the compound represented by formula (1), comprising reacting a dicarboxylic acid represented by formula (4) with a halogenating agent to produce a first acid chloride, reacting the first acid chloride with a hydrazide to produce a first intermediate represented by formula (5) for the blue light-emitting compound, and dehydrating the first intermediate to produce the blue light-emitting compound represented by formula (6).

Formula (4) is: 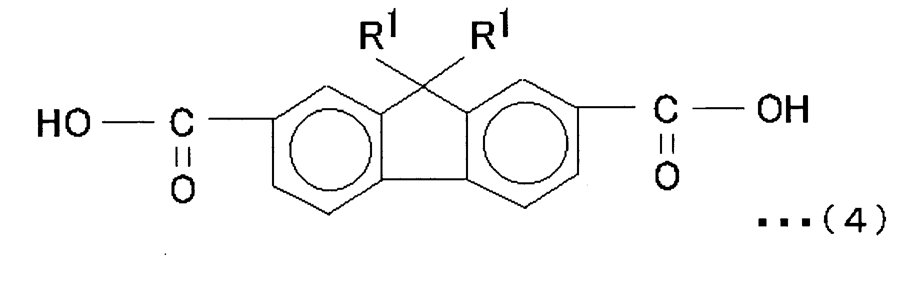 wherein R¹ denotes the same as that defined in relation to the compound represented by formula (1).

Formula (5) is: 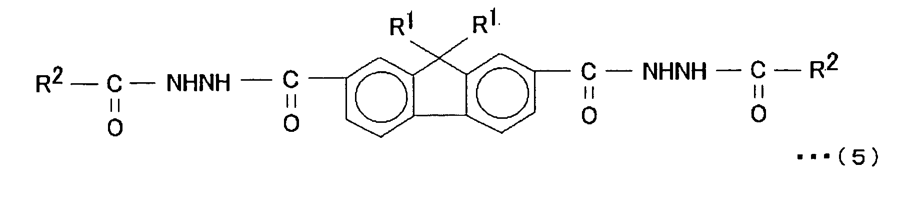 wherein R¹ and R² denote the same as those defined in relation to the compound represented by formula (1).

Formula (6) is: 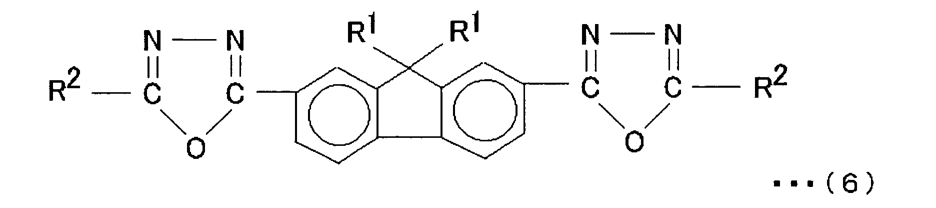 wherein R¹ and R² denote the same as the above-mentioned.

The present invention also provides a process for producing a blue light-emitting compound represented by formula (9), which is another embodiment of the compound represented by formula (1), comprising reacting a carboxylic acid represented by formula (7) with a halogenating agent to produce a second acid chloride, reacting the second acid chloride with a hydrazide to produce a second intermediate represented by formula (8), and dehydrating the second intermediate to produce the blue light-emitting compound represented by formula (9).

Formula (7) is: 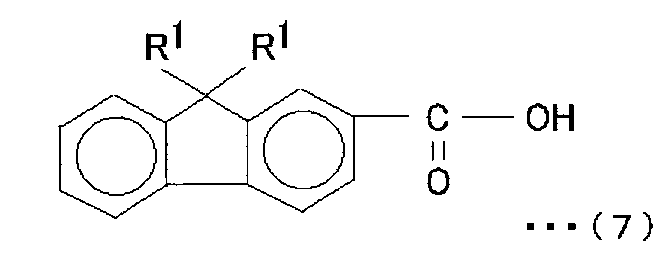 wherein R¹ is the same as that defined in relation to formula (1) .

Formula (8) is: 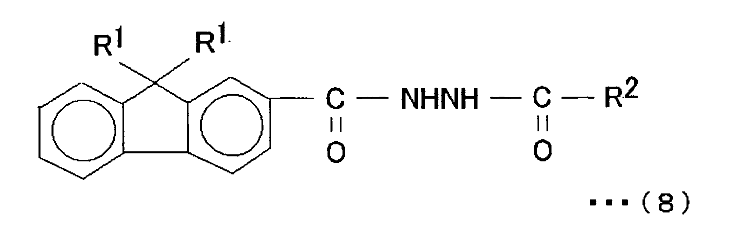 wherein R¹ and R² are the same as those defined in relation to the compound represented by formula (1).

Formula (9) is: 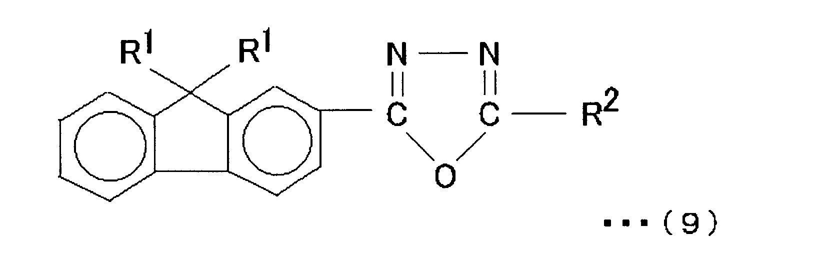 wherein R¹ and R² are the same as those defined above.

The present invention still further provides a process for producing the blue light-emitting compound represented by formula (3), comprising halogenating a fluorene represented by formula (10) to produce a halogenated aromatic compound represented by formula (11), reacting the halogenated aromatic compound with triphenylphosphine to produce an organic phosphoric compound, and reacting the organic phosphoric compound with a carbonyl compound.

Formula (10) is: 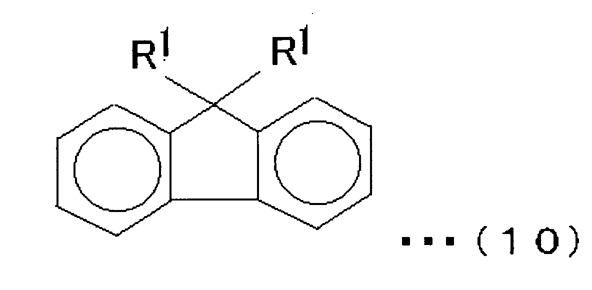 wherein R¹ denotes the same as that defined in relation to the compound represented by formula (1).

Formula (11) is: 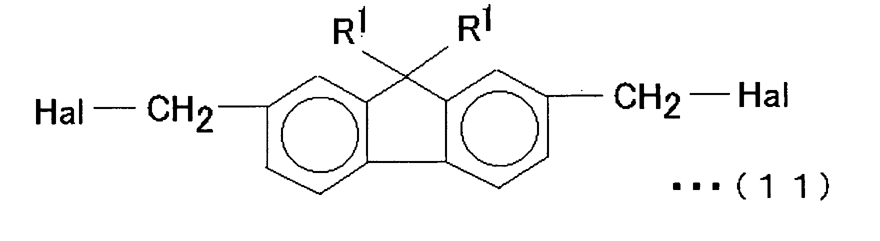 wherein R¹ denotes the same as that defined in relation to the compound represented by formula (1), and "Hal" denotes a halogen atom.

The present invention also provides a luminescent element which has a light-emitting layer including the blue light-emitting compound represented by formula (1) or (3) between a pair of electrodes.

### Brief Description of the Drawings

Figure 1 is an illustration showing an example of the luminescent element in accordance with the present invention.
Figure 2 is an illustration showing another example of the luminescent element in accordance with the present invention.
Figure 3 is an illustration showing a still another example of the luminescent element in accordance with the present invention.
Figure 4 is an illustration showing a further example of the luminescent element in accordance with the present invention.
Figure 5 is an NMR spectrum chart of the blue light-emitting compound synthesized in Example 1.
Figure 6 is an IR spectrum chart of the blue light-emitting compound synthesized in Example 1.
Figure 7 a fluorescence spectrum chart of the blue light-emitting compound synthesized in Example 1.
Figure 8 is an NMR spectrum chart of the blue light-emitting compound synthesized in Example 2.
Figure 9 is an IR spectrum chart of the blue light-emitting compound synthesized in Example 2.
Figure 10 a fluorescence spectrum chart of the blue light-emitting compound synthesized in Example 2.
Figure 11 is an NMR spectrum chart of the blue light-emitting compound synthesized in Example 3.
Figure 12 is an IR spectrum chart of the blue light-emitting compound synthesized in Example 3.
Figure 13 a fluorescence spectrum chart of the blue light-emitting compound synthesized in Example 3.
Figure 14 is an NMR spectrum chart of the blue light-emitting compound synthesized in Example 4.
Figure 15 is an IR spectrum chart of the blue light-emitting compound synthesized in Example 4.
Figure 16 a fluorescence spectrum chart of the blue light-emitting compound synthesized in Example 4.
Figure 17 is an NMR spectrum chart of the blue light-emitting compound synthesized in Example 5
Figure 18 is an IR spectrum chart of the blue light-emitting compound synthesized in Example 5.
Figure 19 a fluorescence spectrum chart of the blue light-emitting compound synthesized in Example 5.
Figure 20 is an NMR spectrum chart of the blue light-emitting compound synthesized in Example 6
Figure 21 is an IR spectrum chart of the blue light-emitting compound synthesized in Example 6.
Figure 22 is an NMR spectrum chart of the blue light-emitting compound synthesized in Example 7
Figure 23 is an IR spectrum chart of the blue light-emitting compound synthesized in Example 7.
Figure 24 is an NMR spectrum chart of the blue light-emitting compound synthesized in Example 8
Figure 25 is an IR spectrum chart of the blue light-emitting compound synthesized in Example 8.
Figure 26 is an NMR spectrum chart of the blue light-emitting compound synthesized in Example 9
Figure 27 is an IR spectrum chart of the blue light-emitting compound synthesized in Example 9.
Figure 28 is a fluorescence spectrum chart of the blue light-emitting compound synthesized in Example 10.
Figure 29 is an NMR spectrum chart of the blue light-emitting compound synthesized in Example 11
Figure 30 is an IR spectrum chart of the blue light-emitting compound synthesized in Example 11.
Figure 31 is a fluorescence spectrum chart of the blue light-emitting compound synthesized in Example 11.
Figure 32 is an NMR spectrum chart of the blue light-emitting compound synthesized in Example 12
Figure 33 is an IR spectrum chart of the blue light-emitting compound synthesized in Example 12.
Figure 34 is a fluorescence spectrum chart of the blue light-emitting compound synthesized in Example 12.
Figure 35 is another fluorescence spectrum chart of the blue light-emitting compound synthesized in Example 12.

### Detailed Description of the Preferred Embodiments

One of the blue light-emitting compounds of the present invention has a chemical structure represented by formula (1) :

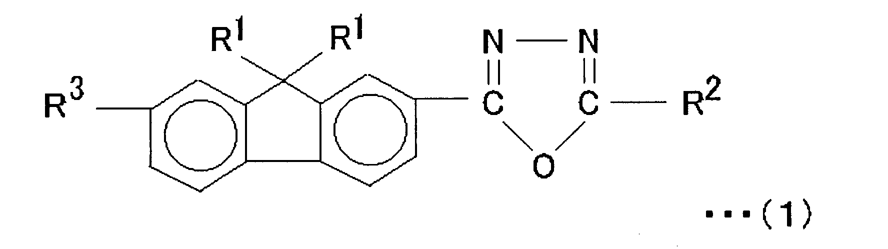

The blue light-emitting compound represented by formula (1) has a structure made of one fluorene skeleton and at least one, preferably two 1,3,4-oxadiazole skeletons.

The 9-positioned carbon atom of the fluorene skeleton is bonded to two R¹s, and the carbon atom of 1,3,4-oxadiazole other than that bonded to the fluorene skeleton is bonded to substituent R².

R¹ denotes an alkyl group with 1 to 15 carbon atoms, a cycloalkyl group with 6 to 15 carbon atoms, or an aryl group represented by formula (1-1), (1-2), (1-3), or (1-4). R² denotes an aryl group represented by formula (1-1), (1-2), (1-3) or (1-4), or a furyl group. Two R¹s may be the same or different from each other.

The alkyl group with 1 to 15 carbon atoms for R¹ includes methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, an undecyl group, a dodecyl group, a tridecyl group, a tetradecyl group, a pentadecyl group etc. Among these is preferred an alkyl group having 1 to 10 carbon atoms, such as methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, or a decyl group. An alkyl group with 1 to 6 carbon atoms is especially preferable.

The cycloalkyl group with 6 to 15 carbon atoms for R¹ includes a cycloalkyl group without substituents and a cycloalkyl group with at least one alkyl group replacing a hydrogen atom thereof.

Formula (1-1) is:

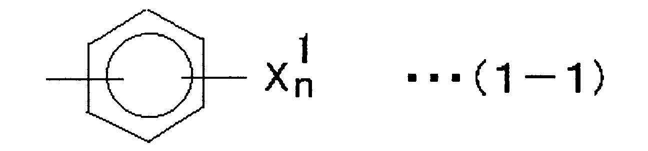

The aryl group represented by formula (1-1) has a phenyl group as its basic skeleton.

X¹ in formula (1-1) denotes an alkyl group with 1 to 10 carbon atoms, a fluorine atom-including alkyl group with 1 to 10 carbon atoms, or a hydrogen atom.

Examples of the alkyl group with 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc. Among these are preferred methyl group, ethyl group, propyl group, isopropyl group, n-butyl group, isobutyl group, sec-butyl group, tert-butyl group, n-pentyl group, sec-pentylgroup, tert-pentylgroup, n-hexylgroup, etc.

The fluorine atom-including alkyl group with 1 to 10 carbon atoms should include at least one fluorine atom. Examples of the group are fluoromethyl group, difluoromethyl group, trifluoromethyl group, fluoroethyl group, 1,1-difluoroethyl group, 1,2-difluoroethyl group, 1,1,1-trifluoroethyl group, 1,1,2-trifluoroethyl group, 1,2,2-trifluoroethyl group, 1,1,2,2-tetrafluoroethyl group, 1,1,2,2,2-pentafluoroethyl group, 1-fluoropropyl group, 2-fluoropropyl group, 1,1-difluoropropylgroup, 1,2-difluoropropyl group, 1,3-difluoro-propylgroup, 2,2-difluoropropylgroup, 1,1,1-trifluoropropyl group, 1,1,2-trifluoropropyl group, 1,2,3-trifluoropropyl group, 1,2,2-trifluoropropyl group, and 1,3,3-trifluoropropyl group. Among these is preferred a fluorine atom-including alkyl group with 1 to 3 carbon atoms.

"n" in formula (1-1) denotes the number of substituents X¹ that can be bonded to the phenyl group. The phenyl group is able to have from 1 to 5 X¹s.

The aryl group may be a group represented by formula (1-2) or (1-3). The numerals 1 to 8 in these formulae mean positions where the hydrogen atoms may be replaced.

Formula (1-2) is:

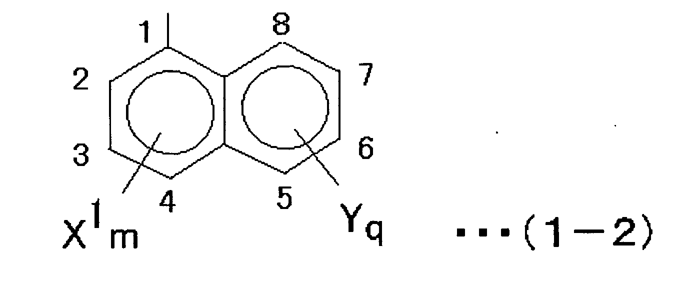

The aryl group represented by formula (1-2) has a naphthyl group as its basic skeleton. At least one of the hydrogen atoms at the 2-, 3-, and 4-positions of the naphthyl group is replaced with X¹, andatleastoneofthoseatthe5-, 6-, 7-, and 8-positions thereof is replaced with Y.

Formula (1-3) is:

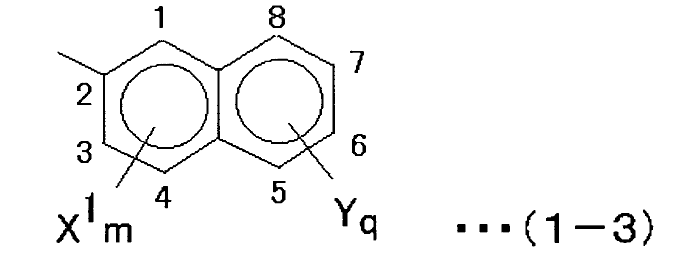

The aryl group represented by formula (1-3) has a naphthyl group as its basic skeleton. At least one of the hydrogen atoms at the 1-, 3-, and 4-positions of the naphthyl group is replaced with X¹, andatleastoneofthoseatthe5-, 6-, 7-, and 8-positions thereof is replaced with Y.

X¹ in formulae (1-2) and (1-3) is the same as that explained in relation to formula (1-1).

"m" in formulae (1-2) and (1-3) denotes the number of substituents X¹ that can be bonded to the respective naphthyl groups. Each naphthyl group is able to have from 1 to 3 X¹s.

Y denotes an alkyl group with 1 to 10 carbon atoms, a fluorine atom-including alkyl group with 1 to 10 carbon atoms, or a hydrogen atom.

Examples of the alkyl group with 1 to 10 carbon atoms are methyl group, ethyl group, propyl group, isopropyl group, n-butyl group, isobutyl group, sec-butyl group, tert-butyl group, n-pentyl group, sec-pentyl group, tert-pentyl group, a hexyl group, a heptyl group, an octyl group, a nonyl group, a decyl group, etc.

The fluorine atom-including alkyl group with 1 to 10 carbon atoms includes fluorine atom-including alkyl groups with 1 to 3 carbon atoms, such as fluoromethyl group, difluoromethyl group, trifluoromethyl group, fluoroethyl group, 1,1-difluoro- ethyl group, 1,2-difluoroethyl group, 1,1,1-trifluoroethyl group, 1,1,2-trifluoroethyl group, 1,2,2-trifluoroethyl group, 1,1,2,2-tetrafluoroethyl group, 1,1,2,2,2-pentafluoroethyl group, 1-fluoropropyl group, 2-fluoropropyl group, 1,1-difluoropropyl group, 1,2-difluoropropyl group, 1,3-difluoropropyl group, 2,2-difluoropropyl group, 1,1,1-trifluoropropyl group, 1,1,2-trifluoropropyl group, 1,2,3-trifluoropropyl group, 1,2,2-trifluoropropyl group, and 1,3,3-trifluoropropyl group.

"q" in formulae (1-2) and (1-3) denotes the number of substituents Y that can be bonded to the respective naphthyl groups. Each naphthyl group is able to have from 1 to 4 Ys.

In formulae (1-2) and (1-3), X¹ and Y may be the same or different from each other.

The aryl group may further be a group represented by formula (1-4) . The numerals 1 to 6 and 1' to 6' show the positions where the hydrogen atoms are replaced.

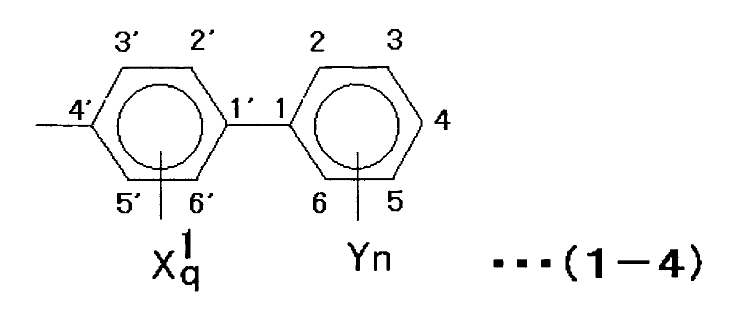

The aryl group represented by formula (1-4) has a biphenyl group as its basic skeleton. At least one of the hydrogen atoms at the 2' -, 3'-, 5'-, and 6'-positions of the group is replaced with X¹, and at least one of those at the 2-, 3-, 4-, 5-, and 6-positions thereof is replaced with Y.

The definitions of X¹, Y, n, and q are the same as those provided in the explanations of formulae (1-1), (1-2), and (1-3) .

X¹ and Y may be the same or different from each other in formula (1-4) as well.

R³ is represented by formula (2): 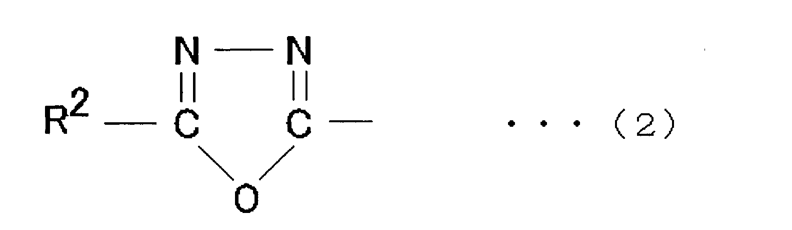 wherein R² denotes the same as that defined above.

Another blue light-emitting compound according to the present invention has a structure represented by formula (3) :

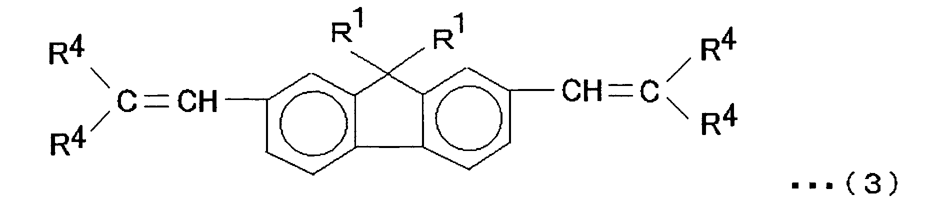

The blue light-emitting compound represented by formula (3) has a basic structure made of one fluorene skeleton and two unsaturated groups having a double bond, -CH=C(R⁴)₂.

The carbon atom at the 9-position of the fluorene skeleton is bonded with two R¹s, and the carbon atom at 2-position of each unsaturated group is bonded with two R⁴s.

R¹ is the same as that explained in relation to formula (1). Two R¹s may be the same or different from each other

R⁴ denotes a hydrogen atom, or an aryl group represented by formula (3-1) or (3-2). Four R⁴s may be the same or different from each other.

Formula (3-1) is: 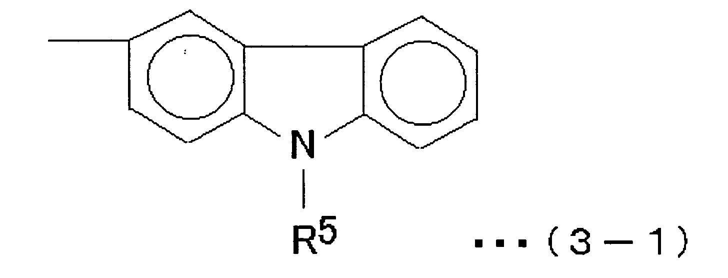 wherein R⁵ denotes a hydrogen atom or an alkyl group with 1 to 5 carbon atoms.

Examples of the alkyl group for R⁵ are methyl group, ethyl group, propyl group, isopropyl group, n-butyl group, isobutyl group, sec-butyl group, tert-butyl group, n-pentyl group, sec-pentyl group, tert-pentyl group, etc. Among these, an alkyl group with 1 to 3 carbon atoms, such as methyl group, ethyl group, propyl group or isopropyl group, is preferable.

Formula (3-2) is: 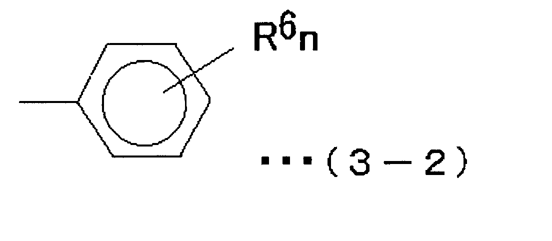 wherein R⁶ denotes a hydrogen atom or an alkyl group with 1 to 5 carbon atoms.

Examples of the alkyl group with 1 to 5 carbon atoms for R⁶ are methyl group, ethyl group, propyl group, isopropyl group, n-butyl group, isobutyl group, sec-butyl group, tert-butyl group, n-pentyl group, sec-pentyl group, tert-pentyl group, etc. Among these, an alkyl group with 1 to 3 carbon atoms, such as methyl group, ethyl group, propyl group or isopropyl group, is preferable.

"n" denotes the number of R⁶s that can be bonded to the phenyl group. The number is from 1 to 5.

In each of the blue light-emitting compounds represented by formulae (1) and (3), R¹ is an electron-donating group, and the bulky aryl groups represented by R² or R⁴ protect the fluorene skeleton from being affected by external conditions. We surmise that this structure makes the π electron cloud thicker and more stable in the basic skeletons of each compound, which enables the compound to emit blue light upon the application of a small amount of energy. The blue light-emitting compounds according to the present invention are characterized by the structure where R¹s, the electron-donating groups, provide the π electron cloud in the basic skeletons of the compounds with electrons. Since these blue light-emitting compounds have stable skeletons, the compounds are chemically stable and will not deteriorate if used under harsh conditions.

The blue light-emitting compound represented by formula (1) may be produced in the following way.

The first step comprises reacting a dicarboxylic acid represented by formula (4) with a halogenating agent. Formula (4) is: 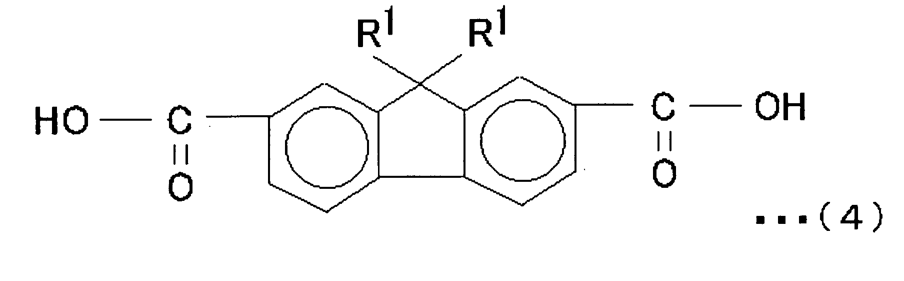 wherein R¹ denotes the same as that defined above.

For the halogenating agent may be employed common agents that are capable of replacing the hydroxyl group of a carboxyl group with a halogen atom. Specific examples of the agent are thionyl chloride, sulfenyl chloride, sulfuryl chloride, phosphorus trichloride, phosphorus pentachloride, hydrogen fluoride,chlorine trifluoride,phosphorustrifluoride,iodine pentafluoride, hydrogen bromide, hypobromous acid, thionyl bromide, etc.

The reaction of the dicarboxylic acid represented by formula (4) with the halogenating agent progresses quickly in a heated solvent. For the solvent may be used acetic anhydride, acetic acid, an acid anhydride having 5 or less carbon atoms, an aromatic hydrocarbon solvent such as benzene or toluene, or 1,4-dioxane. The reaction temperature ranges typically 30°C and 120°C, preferably 60°C and 90°C. After the termination of the reaction, separation and purification by ordinary methods provide a first acid chloride represented by formula (12): 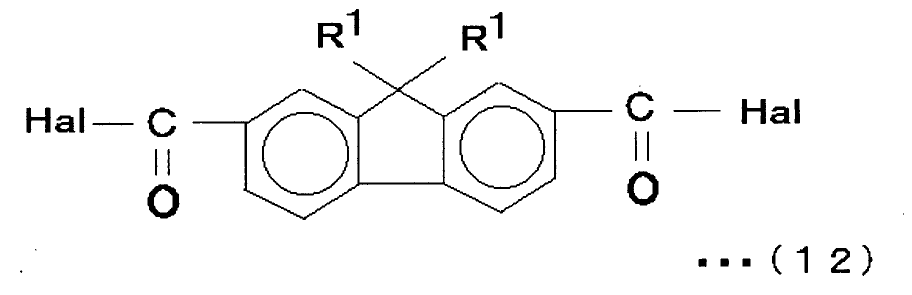 wherein R¹ denotes the same as that explained in relation to formula (1), and "Hal" denotes a halogen atom such as fluorine atom, chlorine atom, bromine atom, or iodine atom.

Then, the first acid chloride represented by formula (12) is reacted with a hydrazide represented by formula (13):

R²-CONHNH₂ (13)

wherein R² is the same as that defined in relation to formula (1) .

The reaction of the first acid chloride with the hydrazide progresses quickly in a heated solvent. The solvent used in this reaction includes, for example, acetic anhydride, acetic acid, an acid anhydride having 5 or less carbon atoms, an aromatic hydrocarbon solvent such as benzene or toluene, 1,4-dioxane, pyridine, and tetrahydrofuran. The reaction temperature is typically from 30°C to 80°C. After the termination of the reaction, a first intermediate for the blue light-emitting compound represented by formula (5) is obtained through purification and separation by ordinary methods. 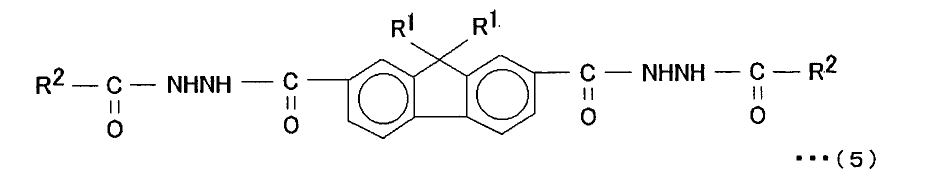 wherein R¹ and R² denote the same as those defined in formula (1) .

A dehydration reaction between the oxygen atoms bonded to the carbon atoms and the hydrogen atoms bonded to the nitrogen atoms takes place when the first intermediate of formula (5) is heated in a solvent. The solvent suitable for the dehydration reaction includes, for example, acetic anhydride, acetic acid, an acid anhydride having 5 or less carbon atoms, an aromatic hydrocarbon solvent such as benzene or toluene, 1,4-dioxane, pyridine, tetrahydrofuran, and phosphoryl chloride. The reaction temperature typically ranges 30°C and 80°C. Separation and purification by ordinary methods after the termination of the reaction provide the blue light-emitting compound according to the present invention.

As understood, the blue light-emitting compound represented by formula (1) of the present invention can easily be producedmerely by heating the first intermediate. The first intermediate itself can easily be made through the simple step of heating the first acid chloride and the hydrazide. Furthermore, the introduction of the halogen atoms into the dicarboxylic acid progresses quickly just by heating. Therefore this simple method of producing the blue light-emitting compound is considered to be an industrial one.

Another blue light-emitting compound represented by formula (9), which is an embodiment of the compound shown by formula (1), may be produced similarly. Formula (9) is: 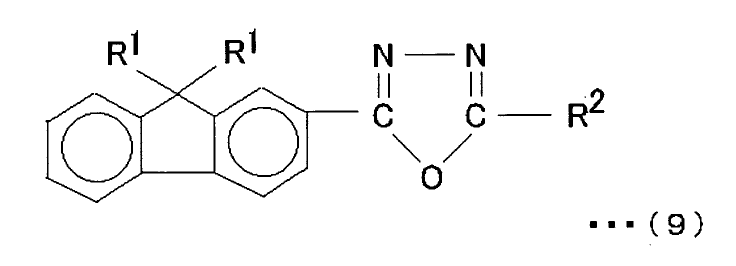 wherein R¹ and R² are the same as those defined hereinbefore.

Specifically, a monocarboxylic acid represented by formula (7) is reacted with a halogenating agent. 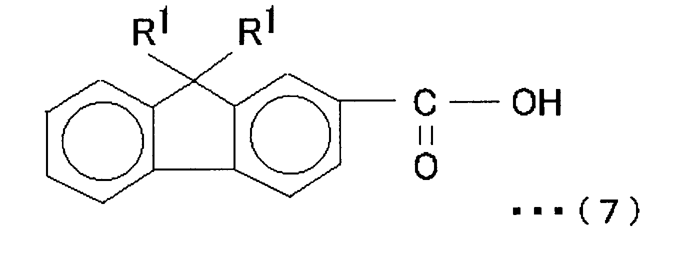 In formula (7), R¹ denotes the same as that defined hereinbefore.

For the halogenating agent may be employed the same ones as those listed for the halogenation of the dicarboxylic acid represented by formula (4).

The monocarboxylic acid represented by formula (7) easily reacts with the halogenating agent under conditions similar to those under which the dicarboxylic acid represented by formula (4) reacts with the halogenating agent. The solvent, reaction temperature, and purification and separation methods for this reaction may be essentially the same as those for the reaction of the dicarboxylic acid. This reaction produces a second acid chloride represented by formula (14): 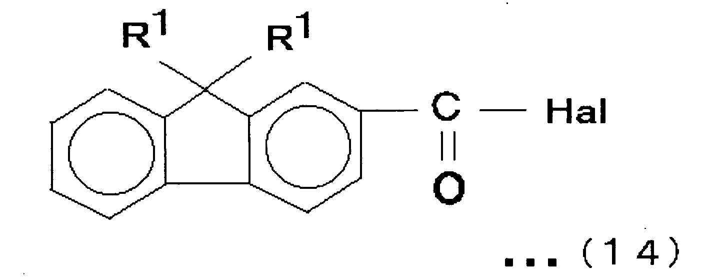 In formula (14), R¹ denotes the same as that defined in formula (1), and "Hal" is a halogen atom, such as fluorine atom, chlorine atom, bromine atom, or iodine atom.

Then, the second acid chloride represented by formula (14) is reacted with the hydrazide represented by formula (13) . The conditions for this reaction are the same as those for the reaction of the acid first chloride represented by formula (12) with the hydrazide represented by formula (13).

Thus, the second intermediate represented by formula (8) is produced.

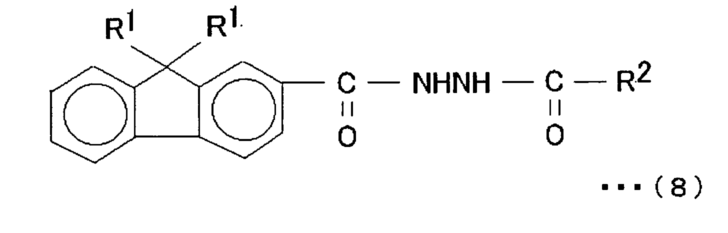

A dehydration reaction between the oxygen atom bonded to one of the carbon atoms and the hydrogen atoms bonded to the nitrogen atoms takes place when the second intermediate of formula (8) is heated in a solvent. The conditions, such as the solvent and the reaction temperature, for this dehydration reaction are the same as those for the dehydration reaction of the first intermediate represented by formula (5).

Separation and purification by ordinary methods after the termination of the reaction provide the blue light-emitting compound of the second intermediate according to the present invention.

The blue light-emitting compound represented by formula (9) of the present invention can easily be produced merely by heating the second intermediate. The second intermediate itself can easily be made through the simple step of heating the second acid chloride and the hydrazide. Furthermore, the introduction of the halogen atom into the monocarboxylic acid progresses quickly just by heating. Therefore this simple method of producing the blue light-emitting compound is considered to be an industrial one.

Also, the blue light-emitting compound represented by formula (3) according to the present invention in the following the way.

Specifically, a fluorene represented by formula (10) is halogenated first.

Formula (10) is: 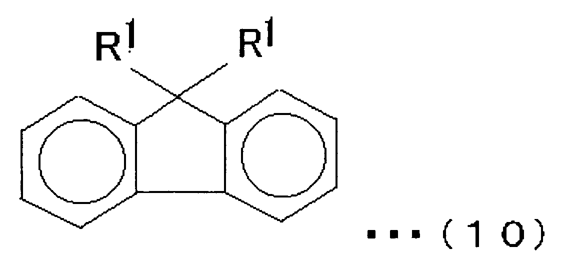 wherein R¹ denotes the same as that defined above.

Then, the fluorene is dissolved in an organic solvent to make a solution. An acid is added to the solution. The obtained acid-including solution is heated, so that a halogenated aromatic compound represented by formula (11) is produced.

Formula (11) is: 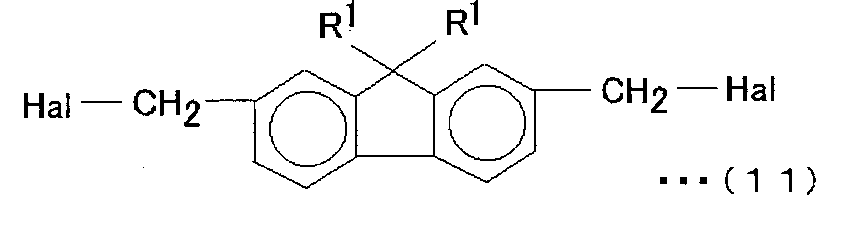 wherein R¹ denotes the same as that defined above, and "Hal" denotes a halogen atom such as a fluorine atom, a chlorine atom, a bromine atom, or an iodine atom.

Examples of the organic solvent that may be used for the reaction are formaldehyde, acetaldehyde, acetic anhydride, acetic acid, glacial acetic acid, benzene, toluene, dioxane, pyridine, tetrahydrofuran, etc.

The acid may be any acid as long as it includes a halogen atom. Examples of the acid are a hydrogen halide such as hydrogen fluoride, hydrochloric acid, hydrogen bromide, or hydrogen iodide; an oxo-acid such as hypochlorous acid, chlorous acid, chloric acid, perchloric acid, hypobromous acid, bromous acid, bromic acid, or perbromic acid.

The reaction temperature is typically from 50°C to 130°C. The halogenated aromatic compound represented by formula (11) can be obtained through separation and purification by ordinary methods after the termination of the reaction.

Then, the halogenated aromatic compound represented by formula (11) and triphenylphosphine (P(C₆H₅)₃) are dissolved in an organic solvent. The obtained solution is heated to make the halogenated aromatic compound react with triphenylphosphine. This reaction is a Wittig reaction.

The organic solvent may be any organic solvent. Examples thereof may be acetic anhydride, acetic acid, an acid anhydride with not more than 5 carbon atoms, an aromatic hydrocarbon solvent such as benzene or toluene, and dioxane. The reaction temperature is typically from 80°C to 130°C. An organic phosphoric compound represented by formula (15) is obtained through separation and purification by ordinary methods after the termination of the reaction.

Formula (15) is: 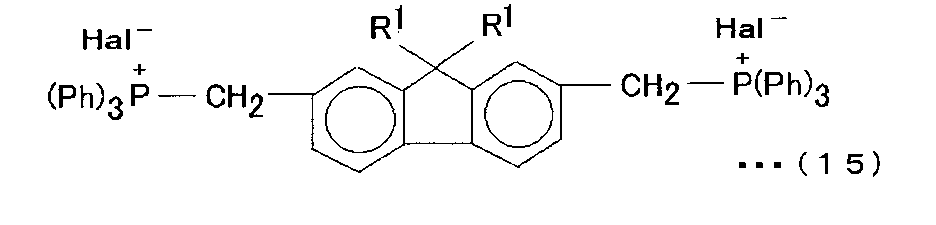 wherein "Ph" means phenyl group.

The organic phosphoric compound represented by formula (15) is mixed with a carbonyl compound represented by formula (16a) or (16b) in a solvent, and the mixture is allowed to react.

R⁴-CH=O (16a)

R⁴-C (=O) -R⁴ (16b)

In formulae (16a) and (16b), R⁴ denotes the same as that defined above. In formula (16b) two R⁴s may be the same or different from each other.

The organic solvent may be any organic solvent. Examples thereof may be acetic anhydride, acetic acid, an acid anhydride with not more than 5 carbon atoms, an aromatic hydrocarbon solvent such as benzene or toluene, dioxane, pyridine, tetrahydrofuran, etc. The reaction temperature is typically from 80°C to 130°C. The blue light-emitting compound represented by formula (3) according to the present invention is obtained through separation and purification by ordinary methods after the termination of the reaction.

The blue light-emitting compound represented by formula (3) of the present invention can easily be produced from the organic phosphoric compound and the carbonyl compound through a Wittig reaction. The organic phosphoric compound can easily be made by the simple step of heating the halogenated aromatic compound and triphenylphosphine. Furthermore, the introduction of the halogen atom into the fluorene to produce the halogenated aromatic compound progresses quickly just by heating. Therefore this simple method of producing the blue light-emitting compound is considered to be an industrial one.

Explanation of the luminescent elements including the blue light-emitting compounds represented by formulae (1) and (3) will be provided hereinafter.

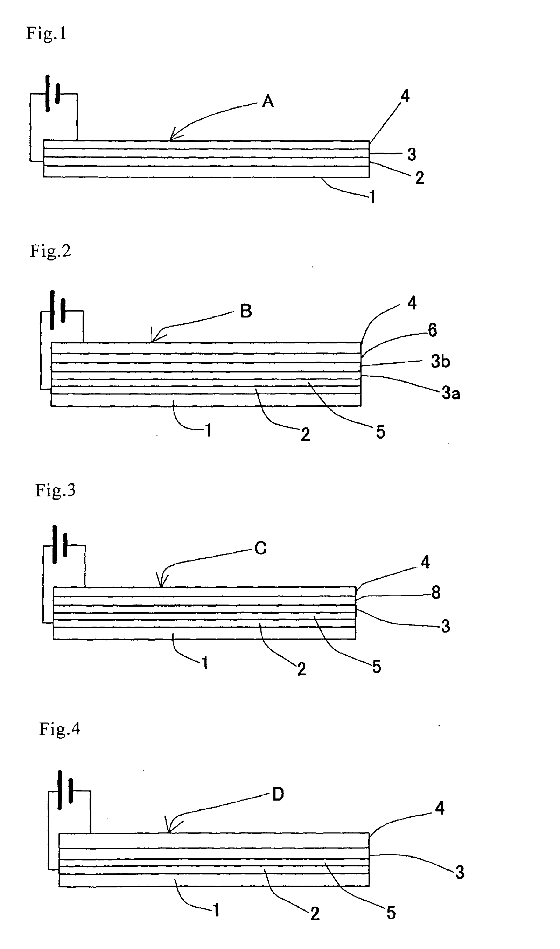

Figure 1 is a schematic illustration that shows the sectional structure of a luminescent element according to the present invention, which is a one-layer type organic EL element. As shown in this figure, the luminescent element A is prepared by layering a light-emitting layer 3 which includes light-emitting compounds and an electrode layer 4 in this order on a substrate 1 with which a transparent electrode 2 has been provided.

When the luminescent element shown in Figure 1 includes a blue light-emitting compound of the present invention, a red light-emitting compound and a green light-emitting compound at a balanced composition, it emits white light upon the application of electricity through the transparent electrode 2 and the electrode layer 4. The total amount of the blue light-emitting compound of the present invention, the red light-emitting compound and the green light-emitting compound, and the proportion of the amount of the blue light-emitting compound to that of the red light-emitting compound to that of the green light-emitting compound, included in the layer 3 to let the element emit white light, vary depending on the kind of each compound. They are decided for each luminescent element depending on the kind of each compound included therein. When the luminescent element is intended to emit blue light, the light-emitting layer 3 may include only a blue light-emitting compound of the present invention. Also, when this luminescent element is intended to emit light of any color other than white and blue, the total amount of the compounds and their respective amounts should be changed depending on the color. For example, when the luminescent element including a blue light-emitting compound of this invention is intended to emit white light, the ratio of the amount of the blue light-emitting compound to that of the red light-emitting compound to that of the green light-emitting compound is usually 5-200:10-100:50-20000 in weight, preferably 10-100:50-500: 100-10000.

For the red-light emitting compound is suitable the Nile Red luminescent compound emitting red light represented by formula (16).

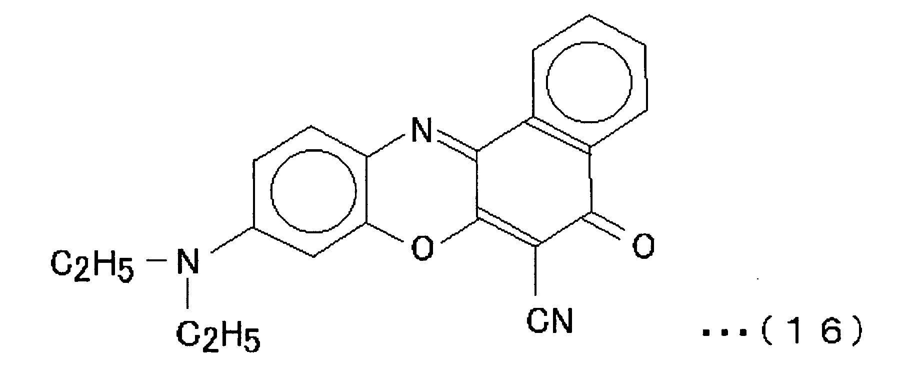

For the green light-emitting compound is suitable a coumarone compound emitting green light, an indophenol compound emitting green light and an indigo compound emitting green light . The coumarin compound represented by the formula (17) is preferable among them.

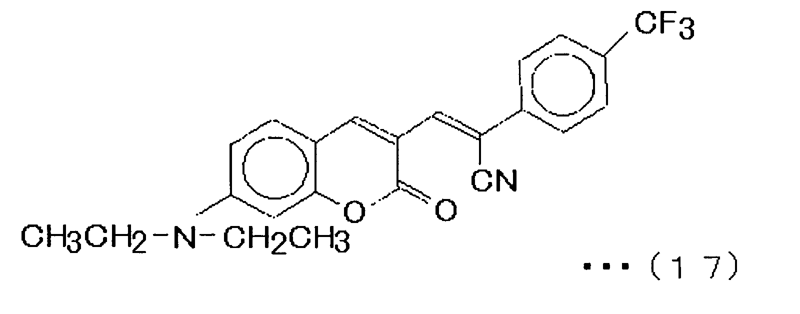

When an electric field is applied between the transparent electrode 2 and the electrode layer 4, electrons are injected from the electrode layer 4 and positive holes are injected from the transparent electrode 2. In the light-emitting layer 3, the electrons are recombined with positive holes, which causes the energy level to return to the valence band from the conduction band. This transition of the energy level is accompanied by emission of the energy differential as light.

The luminescent element A shown in Figure 1, when it is shaped to a planar form with a large area, may be used as a planar illuminator, for example a large-area wall illuminator when fixed on a wall, or a large-area ceiling illuminator when fixed on a ceiling. This luminescent element may be utilized for a planar light source in place of a point light source, such as a conventional bulb, and a line light source, such as a conventional fluorescent lamp. In particular, this illuminator can suitably be used to light up walls, ceilings and floors in dwelling rooms, offices and passenger trains, or to make them emit light. Moreover, this luminescent element A may be suitable for the backlight used in the displays of computers, cellular phones and ATMs. Furthermore, this illuminator may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. In addition, because this luminescent element A includes a blue light-emitting compound of the present invention, which has the specific chemical structure, in the light-emitting layer, the luminescent element A may have a long life. Therefore, light sources employing the luminescent element A will naturally have a long life.

As understood from the foregoing, when the light-emitting layer of the luminescent element A includes a blue light-emitting compound of the present invention and not a red light-emitting compound or a green light-emitting compound, the luminescent element A emits clear blue light.

The luminescent element Amay also be shaped into a tubular light emitter comprising a tubularly shaped substrate 1, a transparent electrode 2 placed on the internal surface of the substrate 1, a light emitting layer 3 and an electrode layer 4 placed on the transparent electrode 2 in this order. Because the luminescent element A does not include mercury, it is an ecological light source and may be a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate whose surface is insulated, for example, by forming thereon a layer of an insulating paint.

When the substrate 1 is opaque, the luminescent element, which includes a red light-emitting compound, a green light-emitting compound and a blue light-emitting compound of the present invention, is a single-faced illuminator that emits white light from one side of the element. On the other hand, when the substrate 1 is transparent, the luminescent element is a double-faced illuminator that emits white light from both of the substrate and the surface layer opposite to the substrate.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 is a layer that includes a blue light-emitting compound according to the present invention when the layer 3 is intended to emit blue light. It includes a red light-emitting compound and a green light-emitting compound in addition to a blue light-emitting compound of the present invention when it is intended to emit white light. The light-emitting layer 3 may be a high polymer film where a blue light-emitting compound according to the present invention, or a red light-emitting compound, a green light-emitting compound and a blue light-emitting compound of the present invention are dispersed in a high polymer. The layer may also be a deposited film prepared by depositing a blue light-emitting compound according to the present invention, or a red light-emitting compound, a green light-emitting compound, and a blue light-emitting compound of the present invention on the transparent electrode 2.

Examples of the high polymer for the high polymer film are a polyvinyl carbazole, a poly(3-alkylthiophen), a polyimide including an arylamide, a polyfluorene, a polyphenylene vinylene, a poly-α-methylstyrene, a copolymer of vinylcarbazole and α-methylstyrene. Among them, a polyvinyl carbazole is preferable.

The amount of the blue light-emitting compound of the present invention, or the total amount of the red light-emitting compound, the green light-emitting compound and the blue light-emitting compound included in the high polymer film is, typically 0.01-2 weight%, preferably 0.05-0.5 weight%.

The thickness of the high polymer film ranges, typically between 30 nm and 500 nm, preferably between 100 nm and 300 nm. When the thickness is too small, the amount of the emitted light may be insufficient. On the other hand, when the thickness is too large, the voltage required to drive the element may be too high, which is not desirable. Besides, the large thickness may reduce the flexibility of the film necessary to be shaped into a planar, tubular, curved, or ring article.

The high polymer filmmay be formedthrough the application of a solution of the high polymer and a blue light-emitting compound of the present invention, or a red light-emitting compound, a green light-emitting compound and a blue light-emitting compound of the present invention dissolved in a suitable solvent. The application method is one selected from a spin cast method, a coating method, a dipping method, etc.

When the light-emitting layer 3 is a deposited film, the thickness of the film is generally 0.1-100 nm, although a preferable thickness is different depending on the structure of layers and other factors. When the thickness is too large or too small, it might cause the same problems as described above.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode layer 4 may easily be formed on the surface of light-emitting layer 3, which, in turn, has been formed on substrate 1, by the technique of metal deposition.

When either of the application or the deposition method is employed, a buffer layer should be inserted between each electrode and the light-emitting layer.

Materials for the buffer layer are, for example, an alkaline metal compound such as lithium fluoride, an alkaline earth metal compound such as magnesium fluoride, an oxide such as an aluminum oxide, and 4,4' -biscarbazole biphenyl (Cz-TPD) . Also, materials for forming the buffer layer between the cathode made of ITO, etc. and the organic layer are, for example, m-MTDATA (4,4',4"-tris(3-methylphenyl-phenylamino)triphenylamine), phthalocyanine, polyaniline, and polythiophene derivatives, and inorganic oxides such as molybdenum oxide, ruthenium oxide, vanadium oxide, and lithium fluoride. When the materials are appropriately selected, these buffer layers can lower the driving voltage of the organic EL element, improve the quantum efficiency of luminescence, and achieve an increase in the luminance of the emitted light.

Next, the second example of the luminescent element according to the present invention is shown in Figure 2. This figure is an illustration showing the sectional layer structure of a luminescent element, which is a multi-layer organic EL element.

As shown in Figure 2, the luminescent element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescent element A in Figure 1.

The light-emitting layer of the luminescent element B comprises light-emitting sublayers 3a and 3b. The light-emitting sublayer 3a is a deposited film formed by depositing a light-emitting compound on the hole-transporting layer 5. The light-emitting sublayer 3b functions as a host material.

Examples of the hole-transporting substance included in the hole-transporting layer 5 are a triphenylamine compound such as N,N'-diphenyl-N,N'-di(m-tolyl)-benzidine (TPD) and α -NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenylyl)-1,3,4-oxadiazole and 2,5-bis (1-naphthyl)-1,3,4-oxadiazole (BND), and2,5-bis(5'-tert-butyl-2'-benzoxazolyl) thiophene. Also, a metal complex material such as quinolinol aluminum complex (Alq3), benzoquinolinol beryllium complex (Bebq2) may be used suitably.

The electron-transporting layer 6 of the luminescent element B shown in Figure 2 includes Alq3 as electron-transporting substance.

The thickness of each layer is the same as that in a known multi-layer organic EL element.

The luminescent element B in Figure 2 functions and emits light in the same ways as the luminescent element A in Figure 1. Therefore, the luminescent element B has the same uses as the luminescent element A.

The third example of the luminescent element of this invention is shown in Figure 3. This figure is an illustration showing the sectional layer structure of a luminescent element, which is a multi-layer organic EL element.

The luminescent element C shown in Figure 3 comprises a substrate 1, and a transparent electrode 2, a hole-transporting layer 5, a light-emitting layer 3, an electron-transporting layer 8, and an electrode layer 4, wherein the transparent electrode and the layers are laid on substrate 1 one by one in this order.

The luminescent element C functions in the same way as the luminescent element B.

Another example of the luminescent element of this invention is shown in Figure 4. The luminescent element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescent elements, other than those shown in Figures 1-4, is a two-layer low molecular weight organic luminescent element having a hole-transporting layer that includes a hole-transporting substance and an electron-transporting luminescent layer that includes a blue light-emitting compound of the present invention laid on the hole-transporting layer, these layers being sandwiched between a cathode, which is the transparent electrode formed on the substrate, and an anode, which is the electrode layer. A specific example of this embodiment is a two-layer pigment-injected luminescent element comprising a hole-transporting layer and a luminescent layer that includes a host pigment and a blue light-emitting compound of this invention as a guest pigment, wherein the luminescent layer is laid on the hole-transporting layer and these layers are sandwiched between the cathode and the anode. Another example is a two-layer organic luminescent element comprising a hole-transporting layer that includes a hole-transporting substance and an electron-transporting luminescent layer that is prepared through co-deposition of a blue light-emitting compound of the present invention and an electron-transporting substance, the latter layer being laid on the former, and these two layers being sandwiched between the cathode and the anode. A specific example of the second embodiment is a two-layer pigment-inj ected luminescent element comprising a hole-transporting layer and an electron-transporting luminescent layer that includes a host pigment and a blue light-emitting compound of this invention as a guest pigment, wherein the luminescent layer is laid on the hole-transporting layer and these layers are sandwiched between the cathode and the anode. A further example is a three-layer organic luminescent element comprising a hole-transporting layer, a luminescent layer including a blue light-emitting compound of this invention that is laid on the hole-transporting layer, and an electron-transporting layer that is laid on the luminescent layer, these layers being sandwiched between the cathode and the anode.

Also, it is preferred if the luminescent layer includes, as a sensitizing agent, rubrene, especially rubrene together with Alq3.

A blue light-emitting element utilizing a blue light-emitting compound of the present invention, or a white light-emitting element utilizing a red light-emitting compound, a green light-emitting compound and a blue light-emitting compound of the present invention may generally be used for an organic EL element driven by direct current, and also by pulses and alternating current.

### Examples

### (Example 1)

### -Synthesis of Blue Light-emitting Compound Represented by Formula (18)-

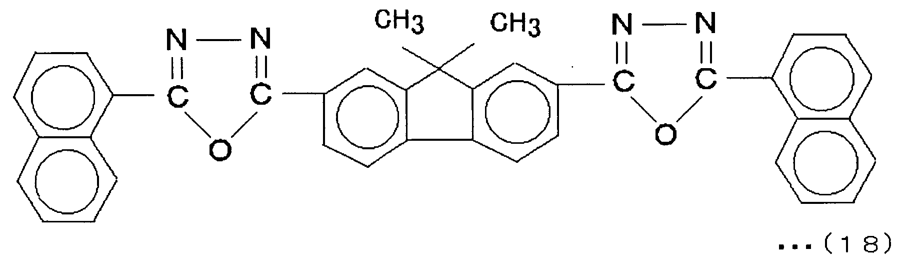

### <Synthesis of a Hydrazide>

In a 1L four-necked flask were placed 23.8 g of 1-naphthoyl chloride, 20 g of anhydrous hydrazine, 12.8 g of pyridine, and 250 ml of tetrahydrofuran. The mixture in the four-necked flask was heated to 50°C in a silicone oil bath and allowed to react for two hours at around the temperature. After the termination of the reaction, the solvent was distilled away with an evaporator. Solids were obtained. A column, which had been filled with silica gel, was charged with the solids, and the solids were purified with chloroform as a developer. 20.06 g of a light yellow solid matter, which was a hydrazide represented by formula (19), was obtained.

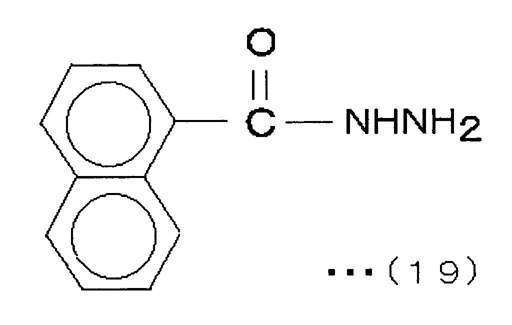

### <Synthesis of an Acid Chloride>

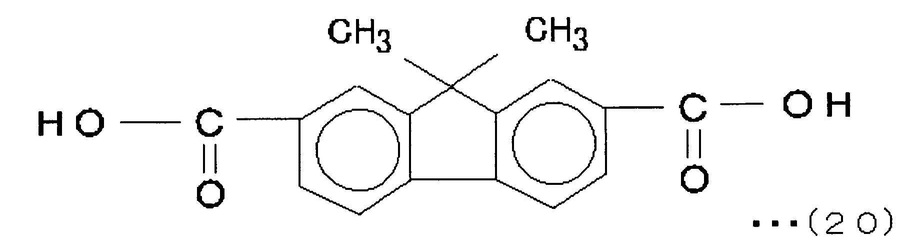

In a 1L pear-shaped flask were placed 20 g of a dicarboxylic acid represented by formula (20) above, 230 ml of 1, 4-dioxane, and 150 ml of thionyl chloride. The solution in the pear-shaped flask was heated in a silicone oil bath to 110°C. Then 40 ml of thionyl chloride was added. The obtained solution was kept at 110°C for 2.5 hours. After the termination of the reaction, the solvent was distilled away with an evaporator. Solids were obtained. A column, which had been filled with silica gel, was charged with the solids, and the solids were purified with chloroform as a developer. 4.9 g of an ocherous solid matter, which was an acid chloride, was obtained.

### <Synthesis of an Intermediate for the Blue Light-emitting Compound>

In a 500 ml four-necked flask were placed 2.7 g of the light yellow solid matter (the hydrazide), 1.55 g of the ocherous solid matter (the acid chloride), 0.9 g of pyridine, and 33 ml of tetrahydrofuran. The reaction mixture in the four-necked flask was heated to 70°C in a silicone oil bath, and allowed to react for 1 hour at around the temperature. After the termination of the reaction, solids in the obtained liquid were separated and washed with water and methanol. The washed solids were dried up, and 2.8 g of an intermediate for the blue light-emitting compound was obtained.

### <Synthesis of the Blue Light-emitting Compound>

In a 300 ml pear-shaped flask were placed 2.8 g of the intermediate, 150 ml of phosphoryl chloride, and 75 ml of 1,4-dioxane. The solution in the pear-shaped flask was heated to 115°C in a silicone oil bath, and allowed to react for 6 hours at around the temperature. After the termination of the reaction, the obtained was introduced into ice water. Precipitates were separated, and washed and neutralized with a 10% aqueous solution of sodium hydroxide. Then, the neutralized precipitates were re-dissolved in benzene, and the resultant was filtered. The filtrate was concentrated and dried up. White crystals with a melting point of 310°C were obtained.

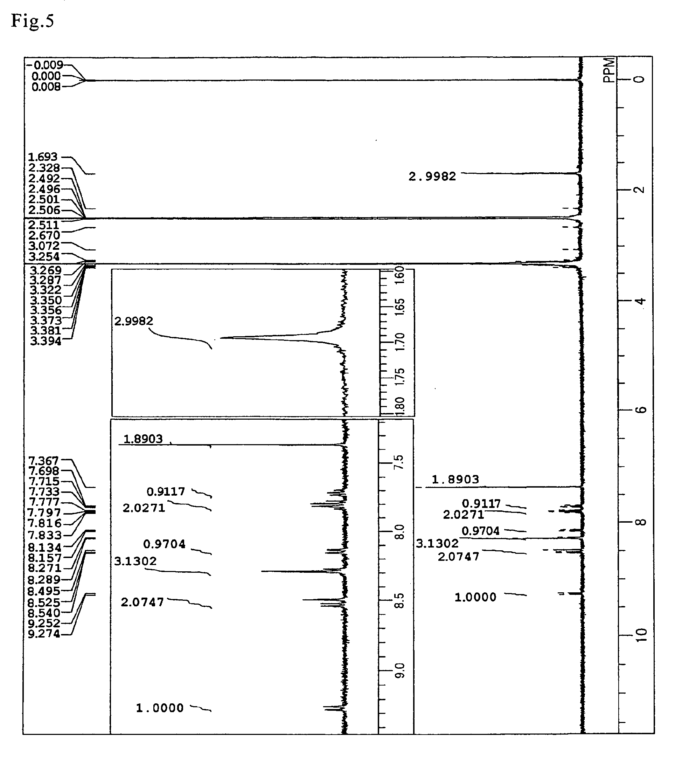

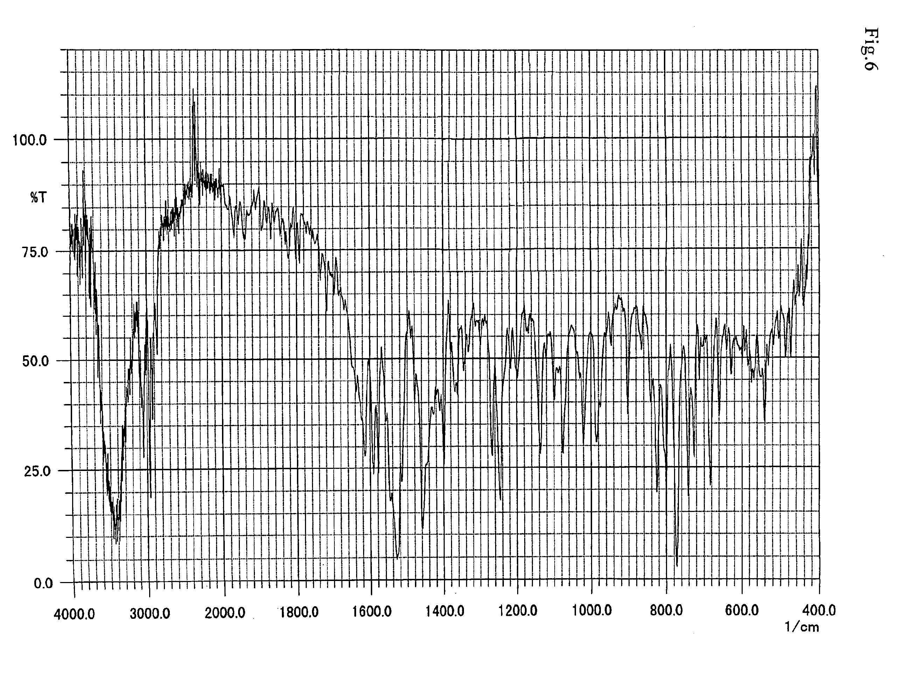

An NMR spectrum chart and an IR spectrum chart of the obtained crystals are respectively shown in Figure 5 and Figure 6. These data confirmed that the crystals produced in this example were the blue light-emitting compound represented by formula (18).

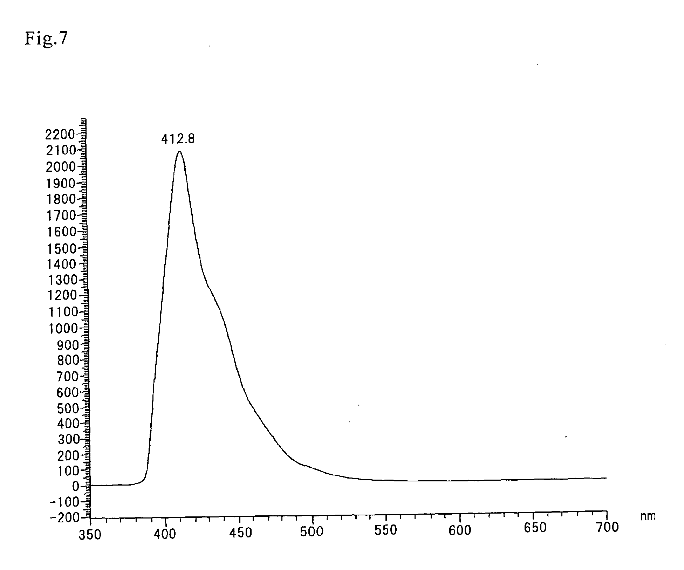

A fluorescence spectrum of the blue light-emitting compound was measured with a model F-45000 spectrofluorophotometer (Exciting wavelength: 365 nm, Solvent: N,N-dimethylacetamide, which may sometimes be abbreviated to "DMAC" hereinafter, Concentration: 0.25% by weight). The wavelength of the maximum emission was 412.8 nm. The measured spectrum is shown in Figure 7.

### <Duration of Luminescence>

Four solutions were prepared by dissolving 5 mg portions of the blue light-emitting compound produced in this example in 10 g of toluene, 5 g of ortho-dichlorobenzene, 2.5 g of tetrahydrofuran, and 2 g of DMAC, respectively. Each solution was irradiated with ultraviolet rays, and the intensity of light emitted by the solution was evaluated by three grades: strong, a little weak, and no luminance. As a result, blue light was emitted strongly for 14 days with the toluene solution, 20 days with the ortho-dichlorobenzene solution, 46 days with the tetrahydrofuran solution, and 100 days or more with the DMAC solution.

### <Luminescent Properties of the Luminescent Element Including the Blue Light-emitting Compound Produced in This Example>

A luminescent element including the blue light-emitting compound produced in this example was prepared, and its luminescent properties were evaluated in the following ways.

An ITO substrate (dimensions: 50 mm x 50 mm; produced bySanyoVacuumIndustriesCo., Ltd.) was ultrasonically cleaned in acetone for 10 minutes, then in 2-propanol for 10 minutes, and dried with nitrogen gas. In addition, the substrate was cleaned through the irradiation of UV rays for 5 minutes with a photo face processor/photo surface processor (produced by SEN Lights Corporation, wavelength: 254 nm).

The cleaned ITO substrate was set in a vacuum metallizer (produced by DIAVAC Limited, model: VDS-M2-46) . α-NPD, the blue light-emitting compound of formula (18) produced in this example, and an aluminum alloy (Al:Li=99:1 (weight ratio), produced by Kojundo Chemical Laboratory Co., Ltd.) were deposited on the substrate in this order under 4 x 10⁻⁶ torr, so that an α-NPD layer with a thickness of 45 nm, a light-emitting layer with a thickness of 40 nm, and an electrode of the aluminum alloy with a thickness of 150 nm were formed on the substrate. Thus, a luminescent element emitting blue light having a layered structure was prepared.

The luminance and the chromaticity of the prepared element were measured with a BM-7 Fast measuring apparatus produced by TOPCON Corporation, with the voltage being raised gradually. The results were that, when the voltage was 14 V and the current was 18.47 mA, the luminance was 3,196.00 Cd/m², chromaticity X 0.2366, and chromaticity Y 0.3025.

### (Example 2)

### -Synthesis of Blue Light-emitting Compound Represented by Formula (21)-

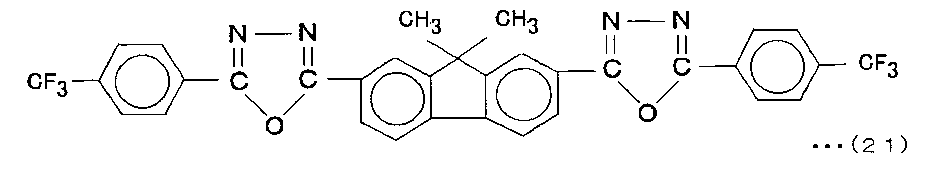

### <Synthesis of a Hydrazide>

In a 500 ml four-necked flask were placed 25 g of 4-trifluoromethylbenzoyl chloride, 38 g of anhydrous hydrazine, 14 g of pyridine, and 70 ml of tetrahydrofuran. The mixture in the four-necked flask was heated to 70°C in a silicone oil bath and allowed to react for 17 hours at around the temperature. After the termination of the reaction, the solvent was distilled away with an evaporator. Solids were obtained. Acolumn, which had been filled with silica gel, was charged with the solids, and the solids were purified with chloroform as a developer. 7.5 g of a white solid matter, which was a hydrazide represented by formula (22), was obtained.

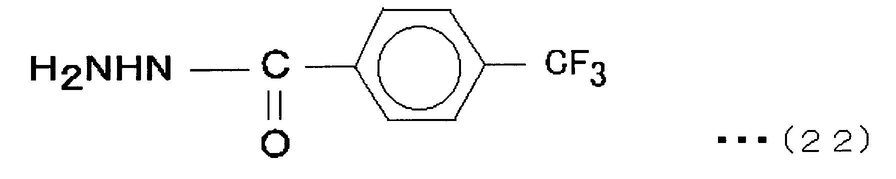

### <Synthesis of an Intermediate for the Blue Light-emitting Compound>

In a 500 ml four-necked flask were placed 2.0 g of the acid chloride obtained in Example 1, 2.8 g of the hydrazide represented by formula (22), 1.2 g of pyridine, and 135 ml of tetrahydrofuran. The reaction mixture in the four-necked flask was heated to 50°C in a silicone oil bath, and allowed to react for 18 hours at around this temperature. After the termination of the reaction, solids in the obtained liquid were separated and washed with water and methanol. The washed solids were dried up, and 2.9 g of an intermediate for the blue light-emitting compound was obtained.

### <Synthesis of the Blue Light-emitting Compound>

In a 300 ml three-necked flask were placed 2.8 g of the intermediate, 65 ml of phosphoryl chloride, and 120 ml of 1,4-dioxane. The solution in the three-necked flask was heated to 110°C in a silicone oil bath, and allowed to react for 9 hours at around this temperature. After the termination of the reaction, precipitates were laid in the bottom. The precipitates were separated and re-dissolved in 150 ml of toluene. The obtained was filtered. The filtrate was concentrated and dried up. 1.12 g of light yellow crystals with a melting point of 288°C was obtained. The yield was 79.6%.

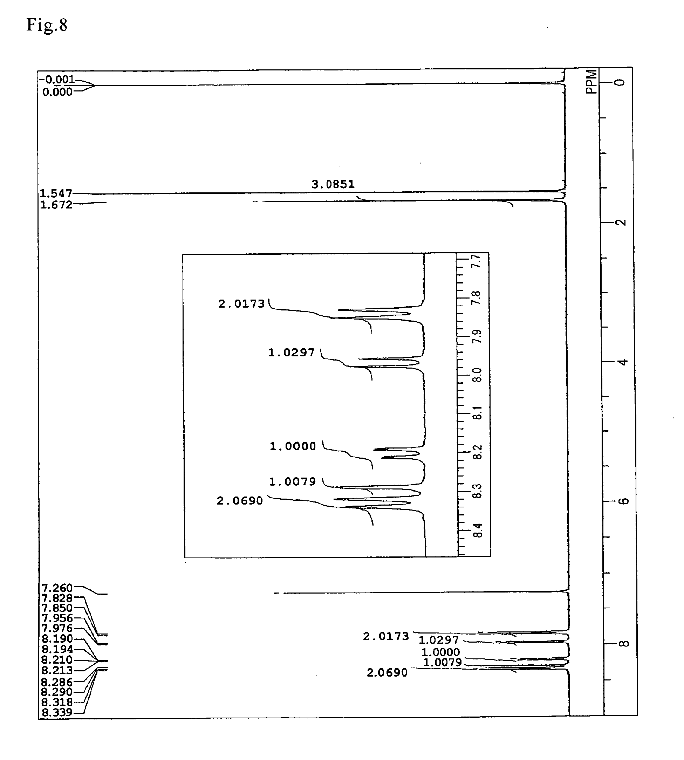

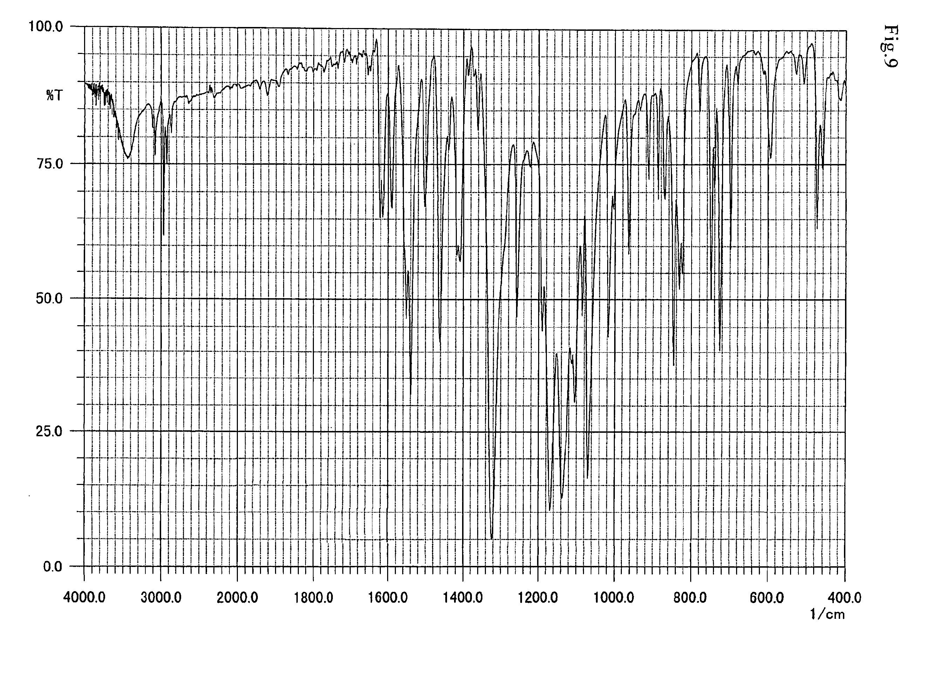

An NMR spectrum chart and an IR spectrum chart of the obtained crystals are respectively shown in Figure 8 and Figure 9. These data confirmed that the crystals produced in this example were the blue light-emitting compound represented by formula (22).

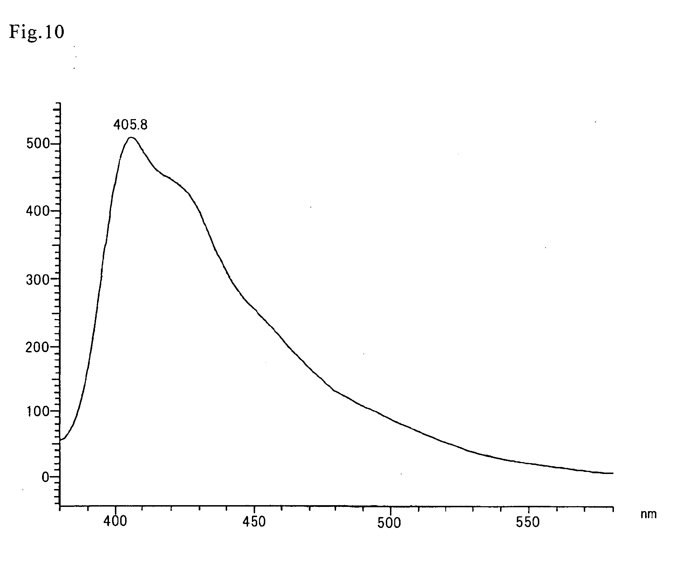

A fluorescence spectrum of the blue light-emitting compound was measured with a model F-45000 spectrofluorophotometer (Exciting wavelength: 365 nm, Solvent: DMAC, Concentration: 0.25% by weight). The wavelength of the maximum emission was 405.8 nm. The measured spectrum is shown in Figure 10.

### (Example 3)

### -Synthesis of Blue Light-emitting Compound Represented by Formula (23)-

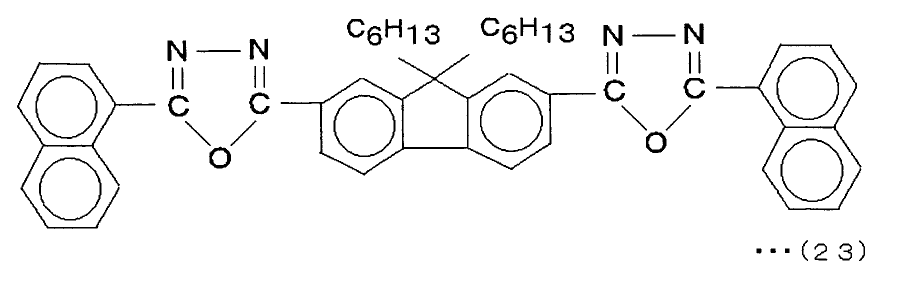

### <Synthesis of a Hydrazide>

1.22 g of the hydrazide represented by formula (19) was prepared. The steps of the preparation were the same as those in Example 1.

### <Synthesis of an Acid Chloride>

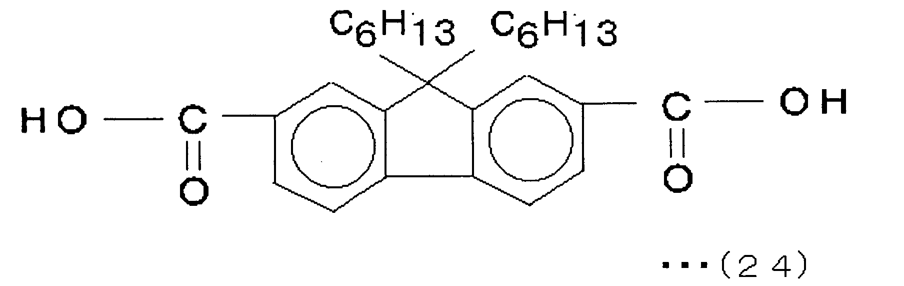

In a 200 ml pear-shaped flask were placed 2.25 g of a dicarboxylic acid represented by formula (24) above and 30 ml of thionyl chloride. The solution in the pear-shaped flask was heated in a silicone oil bath. The solution was kept at 80°C for 1 hour, at 90°C for 0.5 hour, and then at 100°C for 0.5 hour. After the termination of the reaction, solids that were obtained during the reaction were dissolved in 30 ml of tetrahydrofuran. The obtained solution was filtered, so that the filtrate was separated. Then, the solvent was distilled away with an evaporator. 2.0 g of a deep purplish red solid matter, which was an acid chloride, was obtained.

### <Synthesis of an Intermediate for the Blue Light-emitting Compound>

In a 500 ml four-necked flask were placed 1.22 g of the hydrazide, 1 g of the acid chloride, 0.4 g of pyridine, and 15 ml of tetrahydrofuran. The solution in the four-necked flask was heated to 70°C in a silicone oil bath, and allowed to react for 1 hour at around the temperature. After the termination of the reaction, solids in the obtained liquid were separated and washed with water and methanol. The washed solids were dried up, and 1.4 g of a light brown solid matter, which was an intermediate for the blue light-emitting compound, was obtained.

### <Synthesis of the Blue Light-emitting Compound>

In a 500 ml pear-shaped flask were placed 1.4 g of the intermediate and 60 ml of phosphoryl chloride. The solution in the pear-shaped flask was heated to 115°C in a silicone oil bath, and allowed to react for 10 hours at around the temperature. After the termination of the reaction, the product was extracted with chloroform. Distillation of the solvent provided solids. The solids were re-dissolved in 30 ml of a 1:1 mixture of benzene and cyclohexane. The resultant was filtered and the filtrate was separated. The filtrate was concentrated and dried up. Light yellow crystals were obtained.

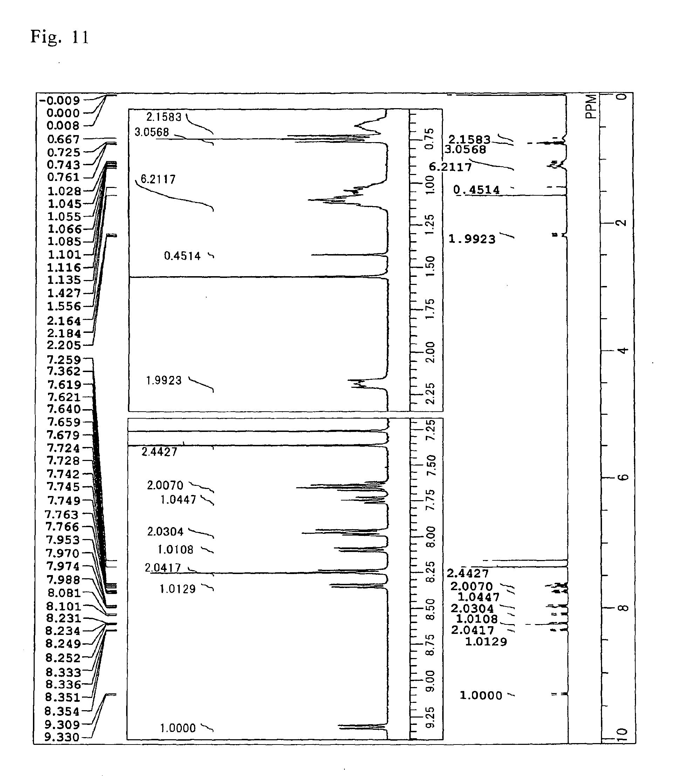

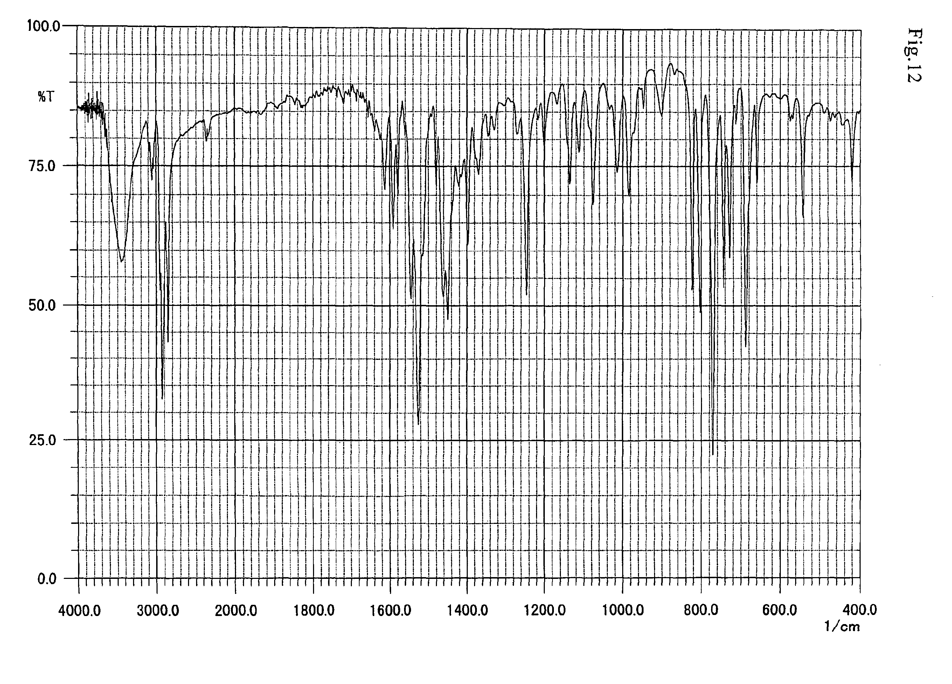

An NMR spectrum chart and an IR spectrum chart of the obtained crystals are respectively shown in Figure 11 and Figure 12. These data confirmed that the crystals produced in this example were the blue light-emitting compound represented by formula (23).

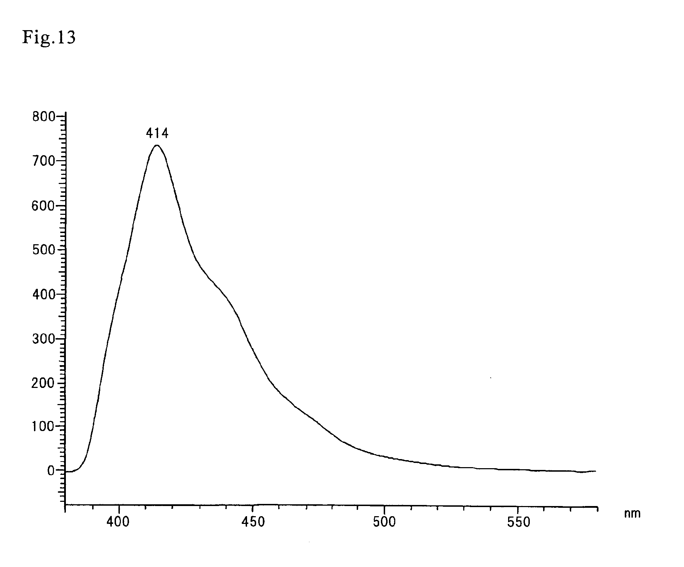

A fluorescence spectrum of the blue light-emitting compound was measured with a model F-45000 spectrofluorophotometer (Exciting wavelength: 365 nm, Solvent: DMAC, Concentration: 0.25% by weight). The wavelength of the maximum emission was 414.0 nm. The measured spectrum is shown in Figure 13.

### (Example 4)

### -Synthesis of Blue Light-emitting Compound Represented by Formula (25)-

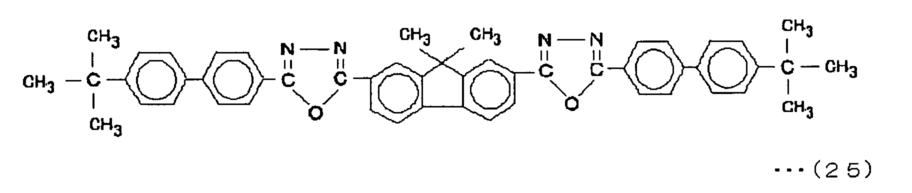

### <Synthesis of a Hydrazide>

In a 300 ml four-necked flask were placed 17 g of 4'-tert-butyldiphenyl acid chloride represented by formula (26) below, 27.7 g of anhydrous hydrazine, 10.3 g of pyridine, and 50 ml of tetrahydrofuran. The obtained mixture in the four-necked flask was heated to 70°C in a silicone oil bath, and allowed to react for 5 hours at around the temperature. After the termination of the reaction, the solvent was distilled away with an evaporator. Solids were obtained. A column, which had been filled with silica gel, was charged with the solids, and the solids were purified with chloroform as a developer. 3 g of a hydrazide was obtained.

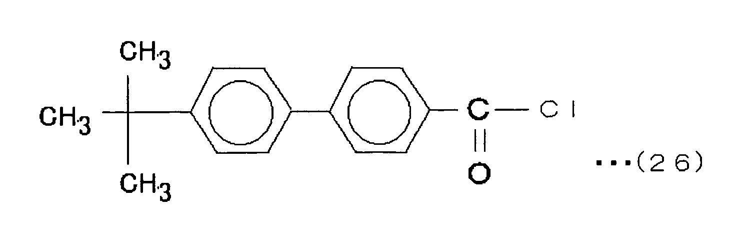

### <Synthesis of an Acid Chloride>

1.62 g of an acid chloride was prepared. The steps of the preparation were essentially the same as those in Example 1.

### <Synthesis of an Intermediate for the Blue Light-emitting Compound>

In a 500 ml four-necked flask were placed 3 g of the hydrazide, 1.62 g of the acid chloride, 0.96 g of pyridine, and 100 ml of tetrahydrofuran. The solution in the four-necked flask was heated to 50°C in a silicone oil bath, and allowed to react for 17 hours at around the temperature. After the termination of the reaction, solids in the obtained liquid were separated and washed with water and methanol. The washed solids were dried up, and 3.8 g of an intermediate for the blue light-emitting compound was obtained.

### <Synthesis of the Blue Light-emitting Compound>

In a 300 ml pear-shaped flask were placed 3.8 g of the intermediate and 30 ml of phosphoryl chloride. The solution in the pear-shaped flask was heated to 110°C in a silicone oil bath, and allowed to react for 14.5 hours at around the temperature. After the termination of the reaction, precipitates that had been formed during the reaction were separated, and washed with chloroform. Then, the washed precipitates were re-dissolved in 300 ml of toluene, and the obtained was filtered. The filtrate was concentrated and dried up. 0.54 g of light yellow crystals with a melting point of 330°C was obtained.

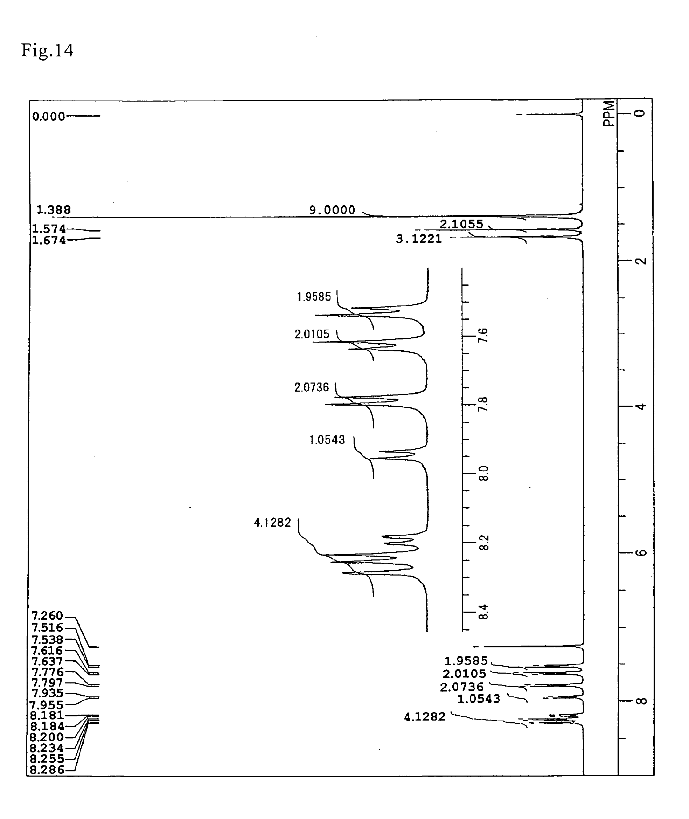

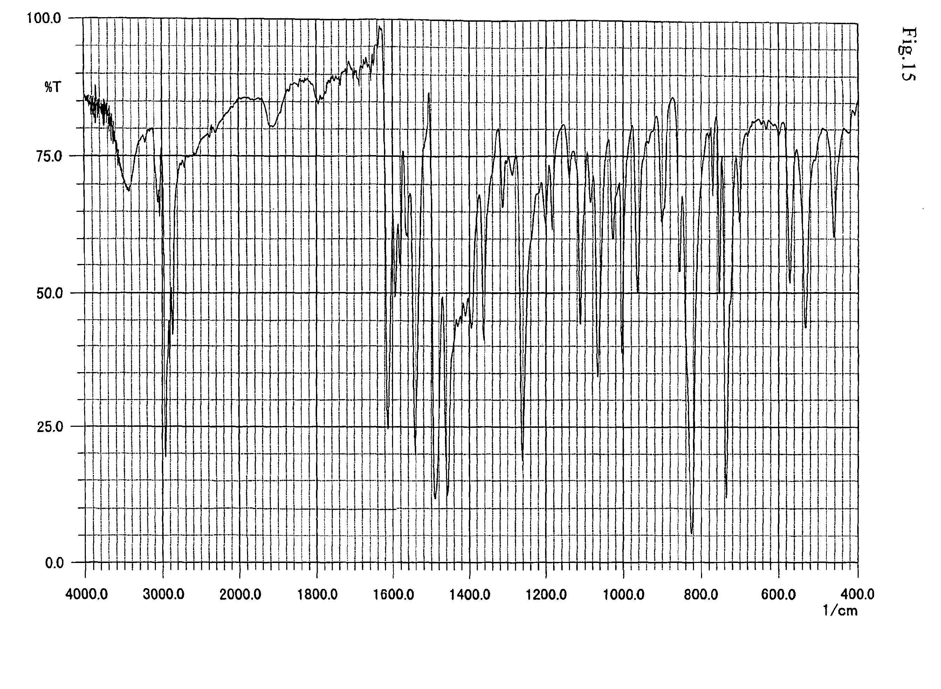

An NMR spectrum chart and an IR spectrum chart of the obtained crystals are respectively shown in Figure 14 and Figure 15. These data confirmed that the crystals produced in this example were the blue light-emitting compound represented by formula (25).

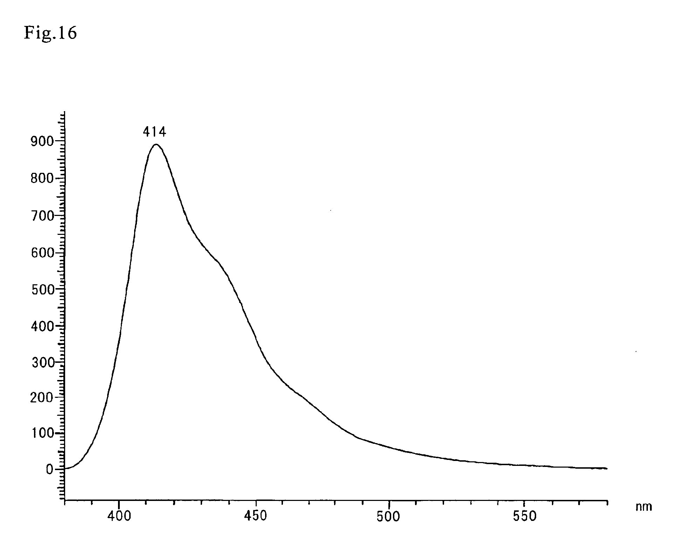

A fluorescence spectrum of the blue light-emitting compound was measured with a model F-45000 spectrofluorophotometer (Exciting wavelength: 365 nm, Solvent: DMAC, Concentration: 0.25% by weight). The wavelength of the maximum emission was 414.0 nm. The measured spectrum is shown in Figure 16.

### (Example 5)

### -Synthesis of Blue Light-emitting Compound Represented by Formula (27)-

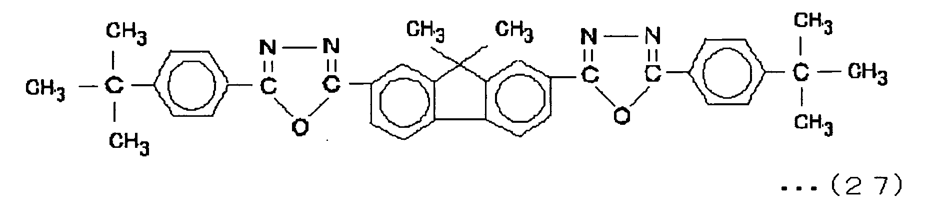

### <Synthesis of a Hydrazide>

In a 300 ml four-necked flask were placed 17 g of 4-tert-butylbenzoyl chloride, 27.7 g of anhydrous hydrazine, 10.3 g of pyridine, and 50 ml of tetrahydrofuran. The mixture in the four-necked flask was heated to 70°C in a silicone oil bath, and allowed to react for 5 hours at around the temperature. After the termination of the reaction, the solvent was distilled away with an evaporator. Solids were obtained. Acolumn, which had been filled with silica gel, was charged with the solids, and the solids were purified with chloroform as a developer. 10.75 g of a hydrazide was obtained.

### <Synthesis of an Acid Chloride>

2 g of an acid chloride was prepared. The steps of the preparation were essentially the same as those in Example 1.

### <Synthesis of an Intermediate for the Blue Light-emitting Compound>

In a 500 ml three-necked flask were placed 2.65 g of the hydrazide, 2 g of the acid chloride, 1.2 g of pyridine, and 50 ml of tetrahydrofuran. The solution in the three-necked flask was heated to 50°C in a silicone oil bath, and allowed to react for 18 hours at around the temperature. After the termination of the reaction, solids in the obtained liquid were separated and washed with water and methanol. The washed solids were dried up, and 3.5 g of an intermediate for the blue light-emitting compound was obtained.

### <Synthesis of the Blue Light-emitting Compound>

In a 500 ml four-necked flask were placed 3.4 g of the intermediate, 65 ml of phosphoryl chloride, and 100 ml of dioxane. The solution in the four-necked flask was heated to 110°C in a silicone oil bath, and allowed to react for 9 hours at around the temperature. After the termination of the reaction, precipitates that had been formed during the reaction were separated, and re-dissolved in 40 ml of toluene. The obtained was filtered. The filtrate was concentrated and dried up. Light yellow crystals were obtained.

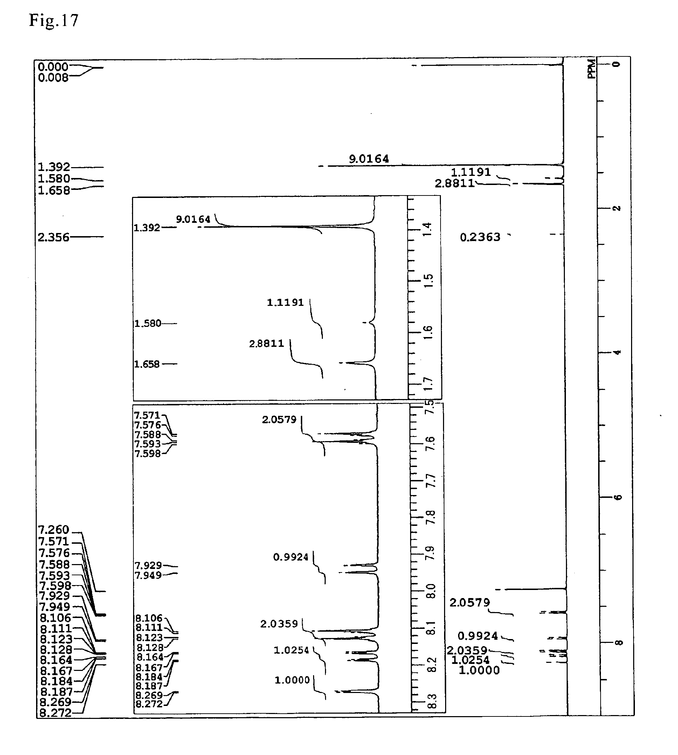

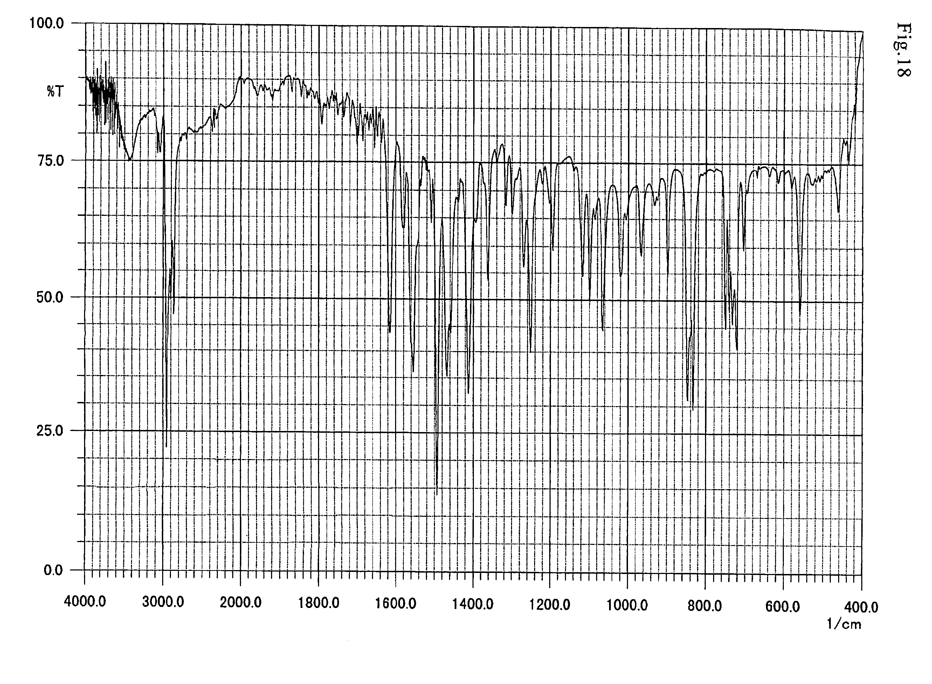

An NMR spectrum chart and an IR spectrum chart of the obtained crystals are respectively shown in Figure 17 and Figure 18. These data confirmed that the crystals produced in this example were the blue light-emitting compound represented by formula (27).

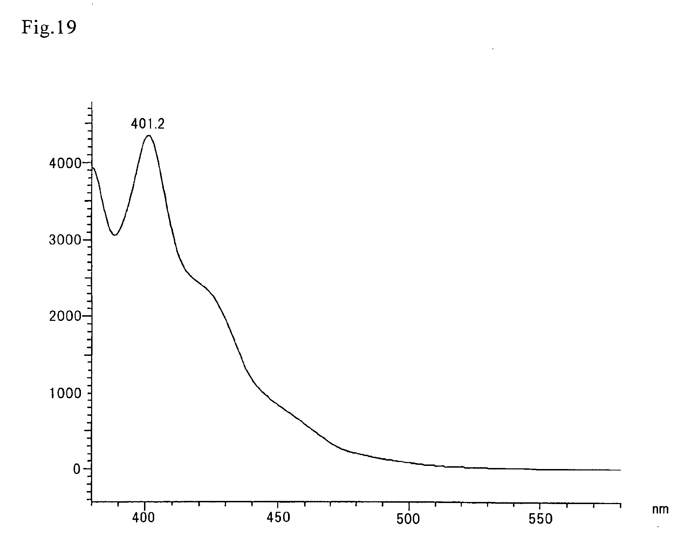

A fluorescence spectrum of the blue light-emitting compound was measured with a model F-45000 spectrofluorophotometer (Exciting wavelength: 365 nm, Solvent: DMAC, Concentration: 0.25% by weight). The wavelength of the maximum emission was 401.2 nm. The measured spectrum is shown in Figure 19.

### (Example 6)

### -Synthesis of Blue Light-emitting Compound Represented by Formula (28)-

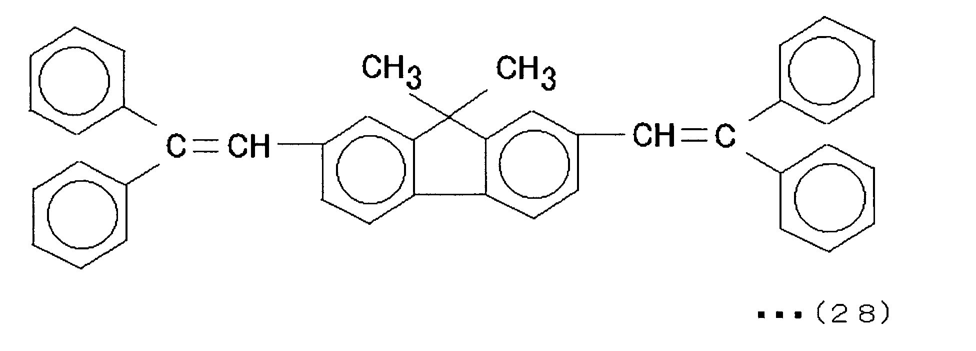

### <Synthesis of a Halogenated Aromatic Compound>

In a 300 ml four-necked flask were placed 2.00 g of 9,9-dimethylfluorene, 12.3 g of a polyphosphoric acid, 1.59 g of formaldehyde, 14.7 g of glacial acetic acid, and 15.2 ml of hydrochloric acid. The mixture in the four-necked flask was stirred vigorously. Then, the mixture in the flask was heated to 115°C in a silicone oil bath, and allowed to react for 7 hours at around the temperature. After the termination of the reaction, the obtained was cooled with ice and filtered. Solids obtained were washed with 300 ml of chloroform, and further washed with 200 ml of pure water. The washed solids were re-dissolved in chloroform, and the solvent was distilled away with an evaporator. 3.1 g of light yellow gel, which was a halogenated aromatic compound, was obtained.

### <Synthesis of an Organic Phosphoric Compound>

In a 200 ml three-necked flask were placed 3.0 g of the halogenated aromatic compound, 8.11 g of triphenylphosphine, and 80 ml of toluene. The mixture in the flask was stirred vigorously. Then the mixture in the flask was heated to 120°C in a silicone oil bath, and allowed to react for a night at around the temperature. After the termination of the reaction, the obtained was cooled with ice and filtered. Solids obtained were washed with 10 ml of benzene. The washed solids were dried in a desiccator. 5.29 g of white crystals were obtained.

### <Synthesis of the Blue Light-emitting Compound>

In a 500 ml pear-shaped flask were placed 1.6 g of the organic phosphoric compound, 0.9 g of benzophenone, and 250 ml of tetrahydrofuran. While the obtained mixture was being cooled with water, 6 ml of n-butyl lithium was dripped into the mixture. The obtained was stirred overnight. Then, the obtained solution was concentrated, the concentrate was extracted with chloroform, and the extract was concentrated again. The concentrated extract was dried up. 3. 87 g of brown crystals were obtained.

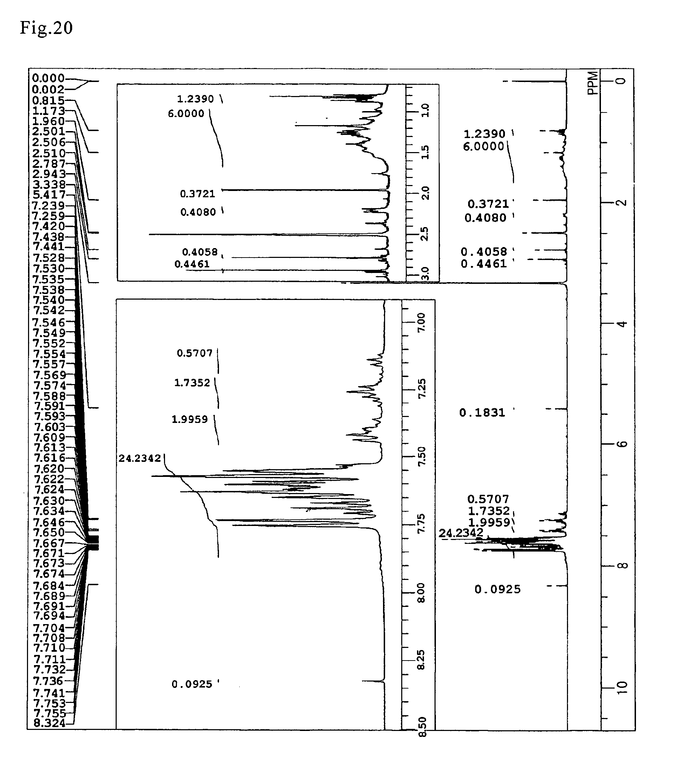

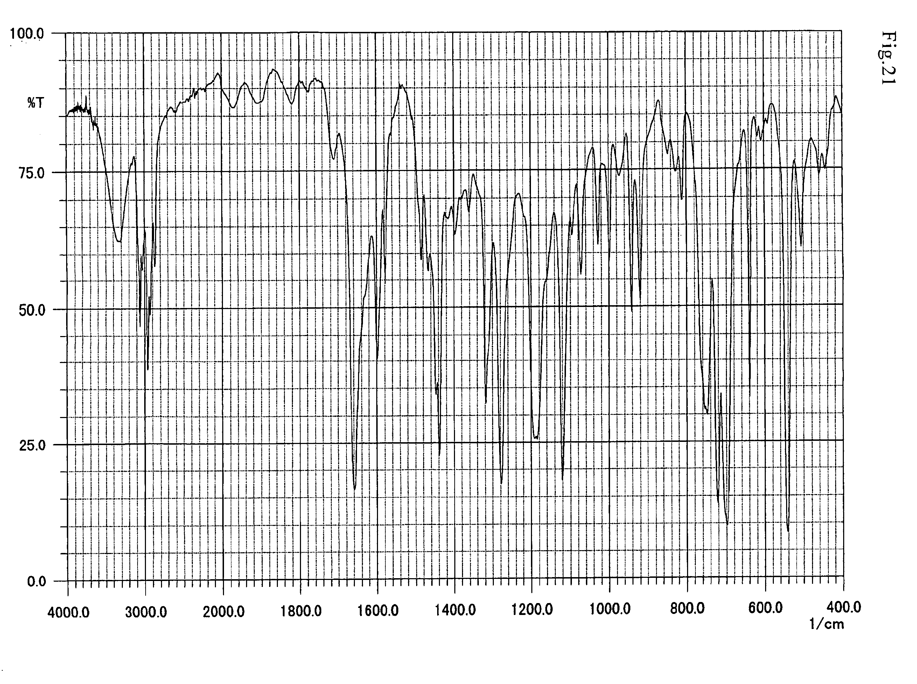

An NMR spectrum chart and an IR spectrum chart of the obtained crystals are respectively shown in Figure 20 and Figure 21. These data confirmed that the crystals produced in this example were the blue light-emitting compound represented by formula (28).

### (Example 7)

### -Synthesis of Blue Light-emitting Compound Represented by Formula (29)-

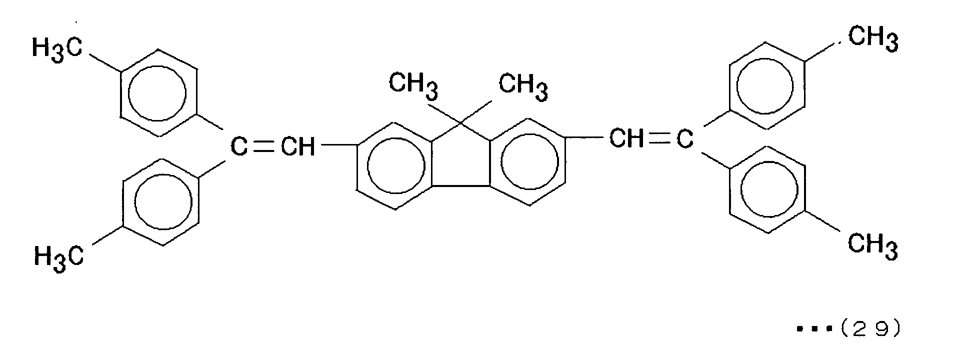

### <Synthesis of the Blue Light-emitting Compound>

In a 500 ml pear-shaped flask were placed 1.1 g of the organic phosphoric compound that had been produced in the same way as in Example 6, 0.85 g of 4,4'-dimethylbenzophenone, and 275 ml of tetrahydrofuran. While the obtained mixture was being cooled with water, 2.88 ml of n-butyl lithium was dripped into the mixture and the resulting mixture was stirred. Then, the resultant was filtered. The solids separated were introduced into 125 ml of chloroform, so that the target compound was dissolved in the chloroform. The chloroform including the target compound was concentrated, and the concentrate was dried up. 1.41 g of yellow crystals was obtained.

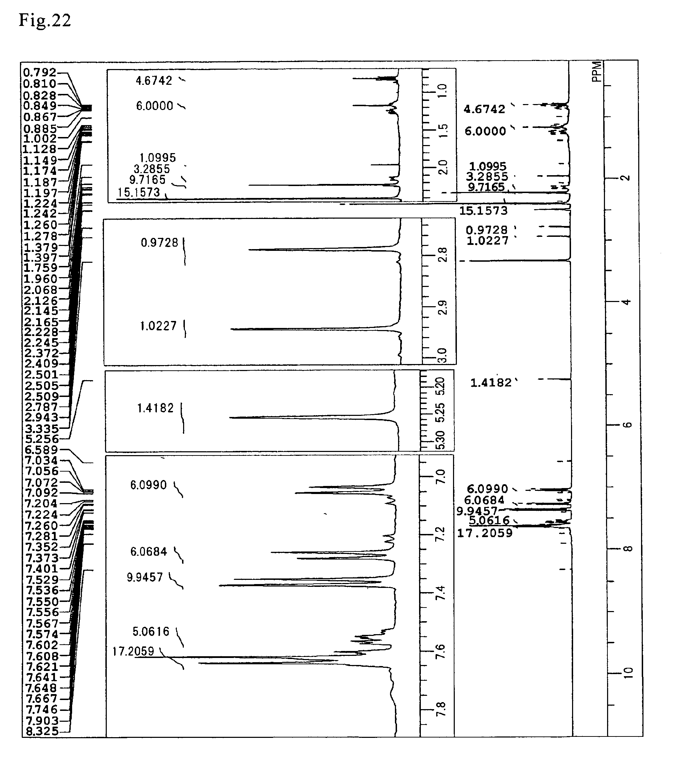

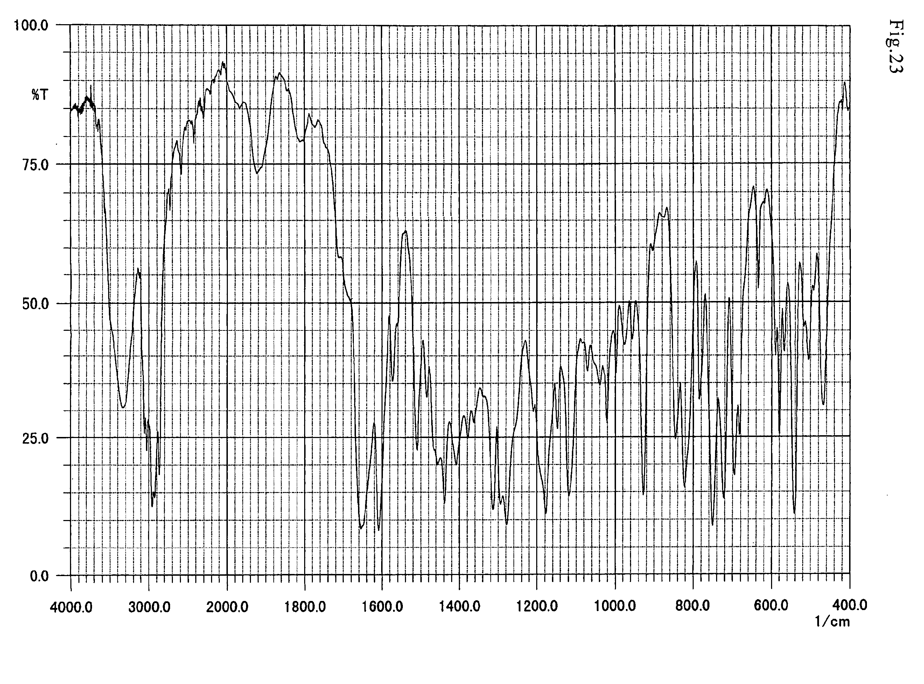

An NMR spectrum chart and an IR spectrum chart of the obtained crystals are respectively shown in Figure 22 and Figure 23. These data confirmed that the crystals produced in this example were the blue light-emitting compound represented by formula (29).

### (Example 8)

### -Synthesis of Blue Light-emitting Compound Represented by Formula (30)-

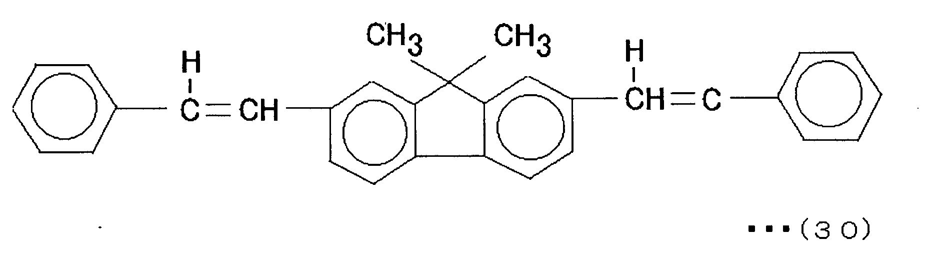

### <Synthesis of the Blue Light-emitting Compound>

In a 500 ml pear-shaped flask were placed 1.3 g of the organic phosphoric compound that had been produced in the same way as in Example 6, 0.16 g of terephthalaldehyde, and 275 ml of tetrahydrofuran. While the obtained mixture was being cooled with water, 3.4 ml of n-butyl lithium was dripped into the mixture and the resulting mixture was stirred. Then, the resultant was filtered. The solids separated were introduced into 125 ml of chloroform, so that the target compound was dissolved in the chloroform. The chloroform solvent was distilled off the target compound with an evaporator. 1.22 g of yellow crystals was obtained.

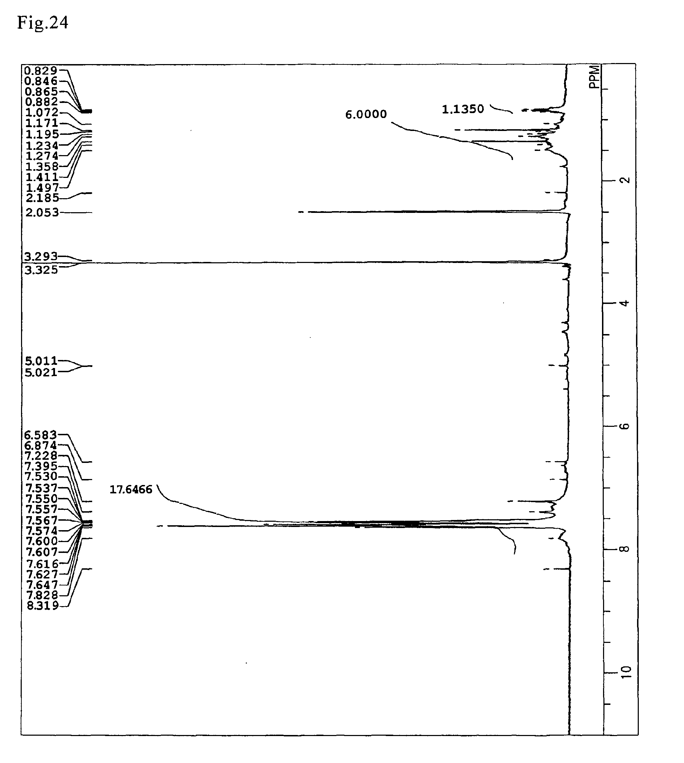

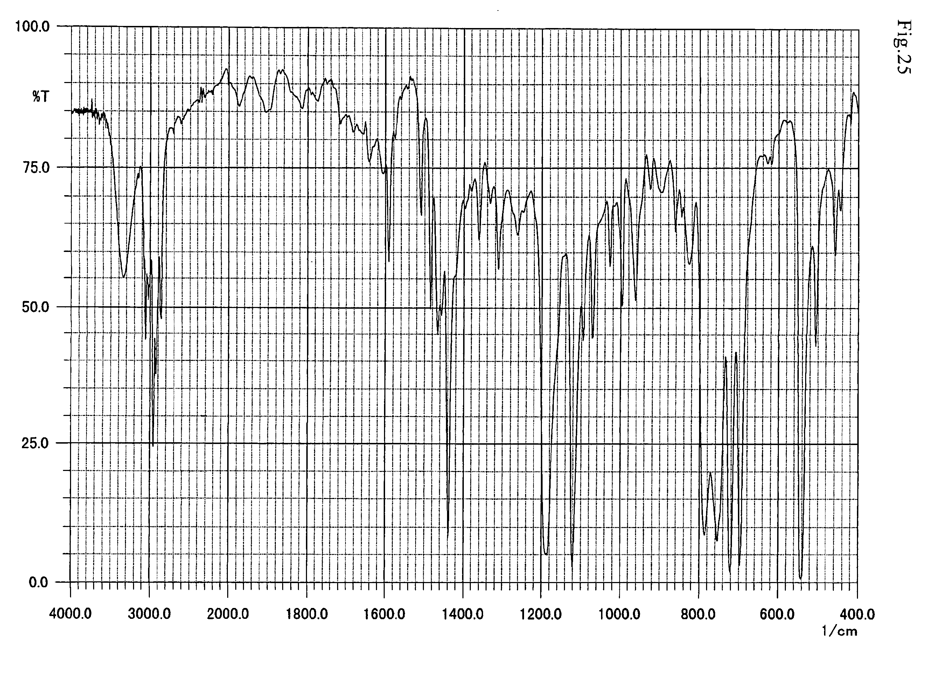

An NMR spectrum chart and an IR spectrum chart of the obtained crystals are respectively shown in Figure 24 and Figure 25. These data confirmed that the crystals produced in this example were the blue light-emitting compound represented by formula (30).

### (Example 9)

### -Synthesis of Blue Light-emitting Compound Represented by Formula (31)-

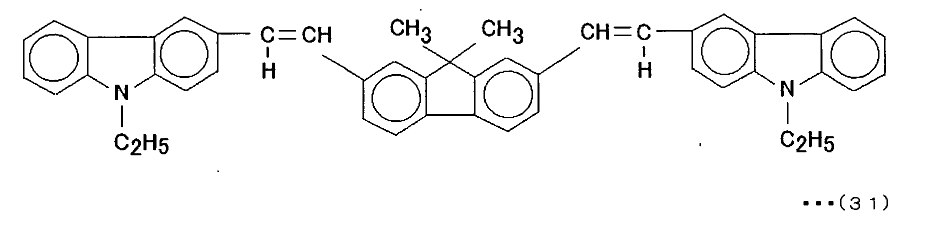

### <Synthesis of the Blue Light-emitting Compound>

In a 500 ml pear-shaped flask were placed 1.3 g of the organic phosphoric compound that had been produced by the same method as in Example 6, 0.82 g of N-ethylcarbazole-3-carboxyaldehyde, and 275 ml of tetrahydrofuran. While the obtained mixture was being cooled with water, 3.4 ml of n-butyl lithium was dripped into the mixture and the resulting mixture was stirred. Then, the resultant was filtered. The solids separated were introduced into 125 ml of chloroform, so that the target compound was dissolved in the chloroform. The chloroform solvent was distilled off the target compound with an evaporator. 2.31 g of reddish brown gel was obtained.

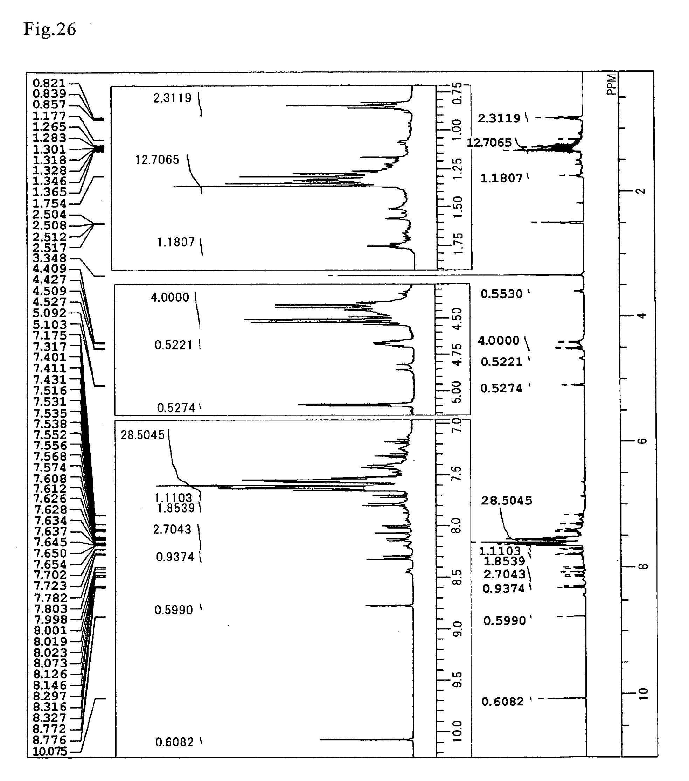

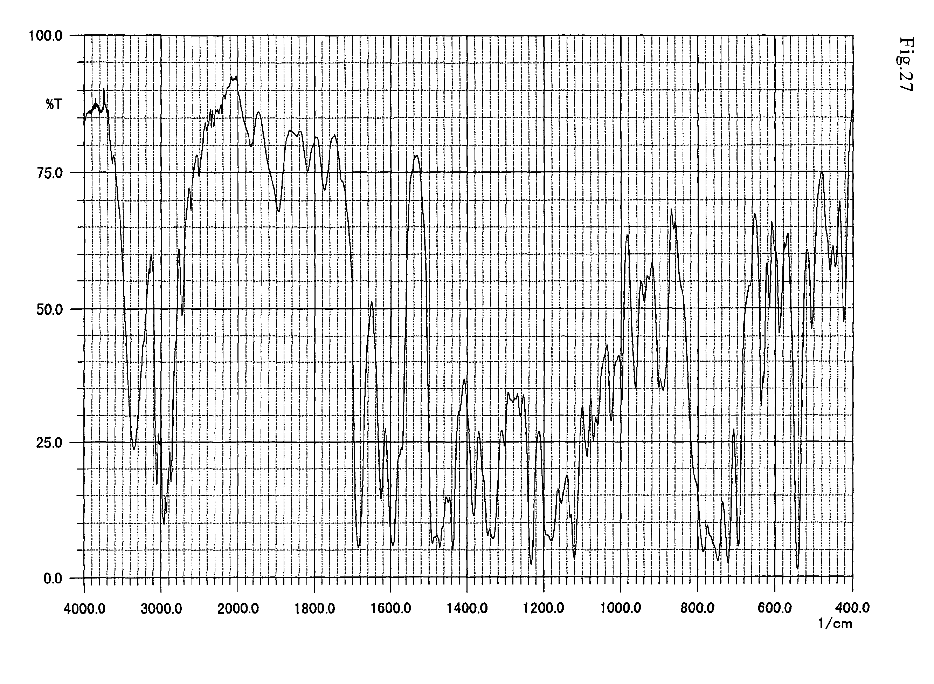

An NMR spectrum chart and an IR spectrum chart of the obtained gel are respectively shown in Figure 26 and Figure 27. These data confirmed that the gel produced in this example were the blue light-emitting compound represented by formula (31) .

### (Example 10)

### -Synthesis of Blue Light-emitting Compound Represented by Formula (32)-

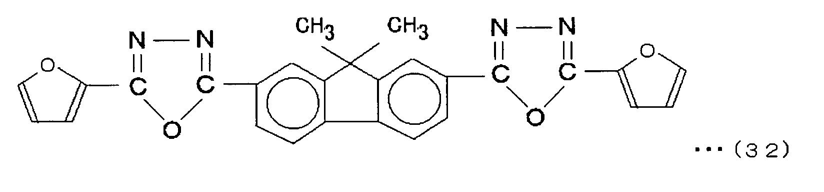

### <Synthesis of 2-furoyl hydrazide>

In a 500 ml three-necked flask were placed 1.34 moles of anhydrous hydrazine and 0.192 mole of pyridine. While the mixture was cooled with ice with stirring, 0.192 mole of 2-furoyl chloride dissolved in 250 ml of tetrahydrofuran was dripped into the mixture gradually. After the completion of the dripping, the resulting mixture in the flask was brought to room temperature. Then the resulting mixture was refluxed at 50°C for about 2 hours. The resultant was brought to room temperature again. Then, the contents in the flask were introduced into ice water. The liquid obtained was extracted with about 500 ml of chloroform. The chloroform solvent was distilled away, and 2.78 g of yellow viscous liquid, which was 2-furoyl hydrazide represented by formula (33), was obtained.

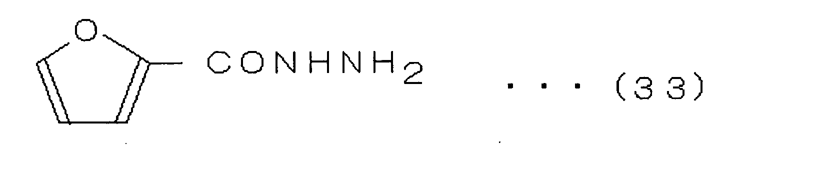

### <Synthesis of an Acid Chloride>

The acid chloride derived from the dicarboxylic acid represented by formula (20) was prepared. The steps of the preparation were the same as those in Example 1.

### <Synthesis of an Intermediate for the Blue Light-emitting Compound>

In a 300 ml three-necked flask were placed 1.102 × 10⁻² mole (1.76 g) of 2-furoyl hydrazide and 1.10 × 10⁻² mole (0.86 g) of pyridine. While the mixture was cooled with ice with stirring, 5.61 x 10⁻³ mole (1.76 g) of 9,9-dimethylfluorene-2,7-dicarbonyl chloride dissolved in 100 ml of tetrahydrofuran was dripped into the mixture gradually. After the completion of the dripping, the resulting mixture in the flask was brought to room temperature. Then the resulting mixture was refluxed for about 2 hours while being heated and kept at 85°C. After brought to room temperature again, the resultant was concentrated and dried up. The obtained solids were washed with water and further with methanol, and dried. The intermediate represented by formula (34) was obtained.

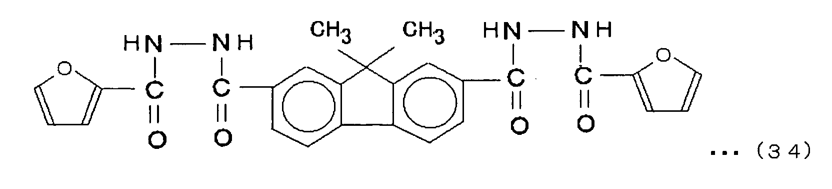

### <Synthesis of the Blue Light-emitting Compound>

The intermediate represented by formula (34) was introduced into about 100 ml of phosphorus oxychloride. The resultant was heated and refluxed overnight under a nitrogen atmosphere. The product was brought to room temperature and introduced into ice water. The obtained was extracted with chloroform, and the chloroform was distilled off the extract. 0.90 g of the target compound represented by formula (32) was obtained.

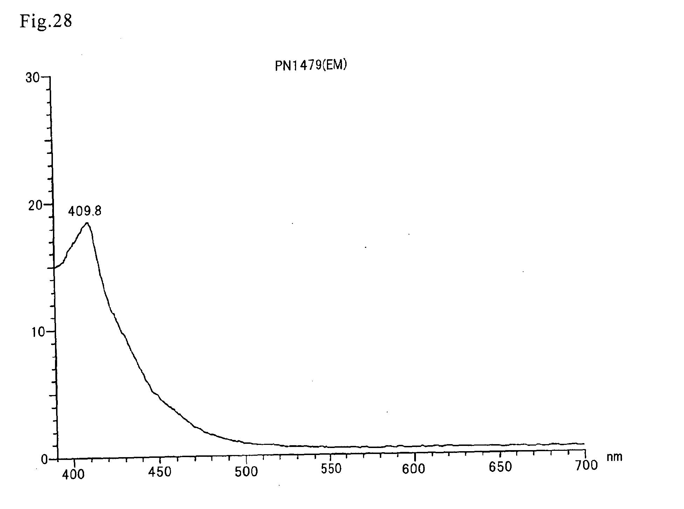

A fluorescence spectrum of the blue light-emitting compound represented by formula (32) was measured with a model F-45000 spectrofluorophotometer (Exciting wavelength: 365 nm, Solvent: dioxane, Concentration: 0.25% by weight). The wavelength of the maximum emission was 409.8 nm. The measured spectrum is shown in Figure 28.

### (Example 11)

### -Synthesis of Blue Light-emitting Compound Represented by Formula (35)-

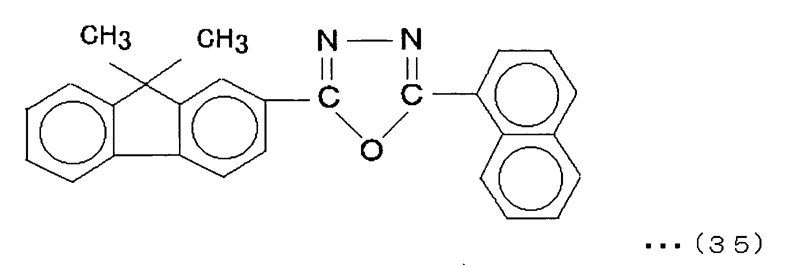

### <A Friedel-Crafts Reaction>

### -Synthesis of a Ketone Represented by Formula (36)-

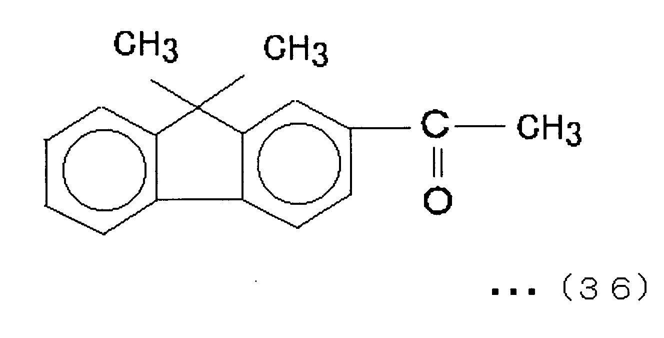

In a 500 ml three-necked flask were placed 1 mole (25 g) of fluorene, 1.1 moles (19 g) of aluminum trichloride and 200 ml of carbon disulfide. The contents in the flask were gradually heated to 60°C, and refluxed for 2 hours at the temperature. Then, the reaction product was introduced into ice water. The resultant was extracted with chloroform, and the solvent was distilled off the extract with an evaporator. The extract was vacuum dried at 45°C. 30.85 g of a peach-colored viscous substance, which was the ketone represented by formula (36), was obtained.

### <Synthesis of an Acid Halide>

In a 2000 ml three-necked flask were placed 16.5 g of the peach-colored substance and 250 ml of methanol. Then, 500 ml of an aqueous solution of sodium hypochlorite (effective chlorine amount: 5%) was poured into the flask. The mixture in the flask was heated and kept at temperatures of 65-90°C for 3.5 hours. After the termination of the heating, the reaction product was cooled to room temperature and filtered. To the obtained filtrate was added a concentrated hydrochloric acid, which resulted in the formation of white precipitates.

The filtrate including the precipitates was filtered with a glass filter. The precipitates were collected, and vacuum dried overnight. 7.5 g of a monocarboxylic acid represented by formula (37) was obtained.

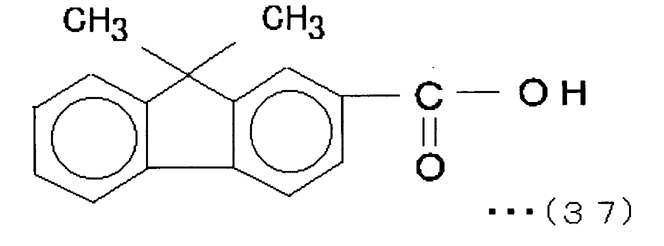

10 g (4.2 × 10⁻² mole) of the monocarboxylic acid represented by formula (37) and 75 ml of thionyl chloride were placed in a 500 ml pear-shaped flask. The contents in the flask were heated and refluxed for 2 hours at 110°C. Then, the reaction product was cooled gradually, and concentrated with an aspirator.

The concentrate was introduced into tetrahydrofuran, and the resultant was filtered. The filtrate was subjected to vacuum distillation with a vacuum pump, and the solvent was removed. The collected residue was allowed to stand in a refrigerator. An acid chloride represented by formula (38) was obtained.

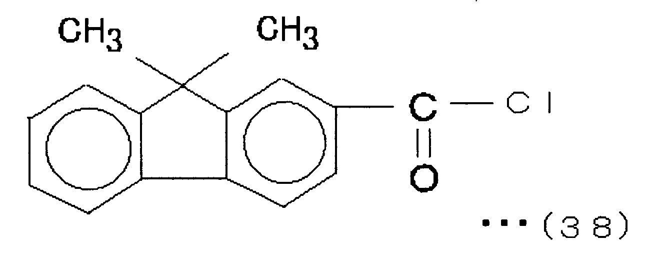

### <Synthesis of an Intermediate for the Blue Light-emitting Compound>

3.5 g (1.37 × 10⁻² mole) of the acid chloride represented by formula (38), 2.55 g of the hydrazide represented by formula (19), and 150 ml of tetrahydrofuran were placed in a 500 ml three-necked flask. The mixture in the flask was heated to 70°C in a silicone oil bath and allowed to react for 1 hour at around the temperature. After the termination of the reaction, the reaction product was introduced into ice water. The resultant was extracted with 900 ml of chloroform. The chloroform solution was washed twice with 200 ml each of water. The solution was dried with anhydrous sodium sulfate, and filtered. The solvent was removed with an evaporator. 5.5 g of a white solid matter, which was an intermediate represented by formula (39), was obtained.

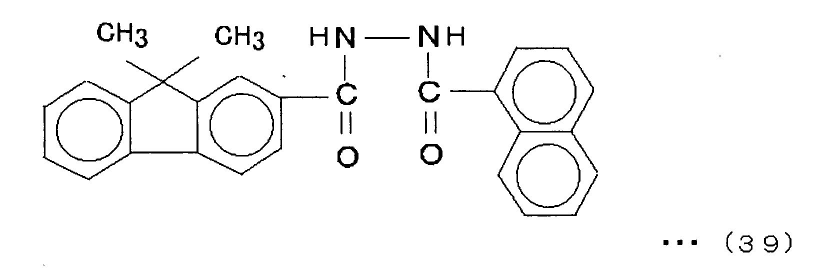

### <Synthesis of the Blue Light-emitting Compound>

5.0 g of the intermediate represented by formula (39), 120 ml of dioxane, and 150 ml of phosphorus oxychloride were placed in a pear-shaped flask. The mixture was refluxed for 10 hours at 115°C. Then, the reaction product was cooled and introduced into ice water. The resultant was extracted with chloroform, which was followed by drying the chloroform solution. The solvent was removed off the extract with an evaporator. Finally, the extract was vacuum dried. 4.6 g of the target compound represented by formula (35) above, the melting point of which ranged between 175°C and 181°C, was obtained.

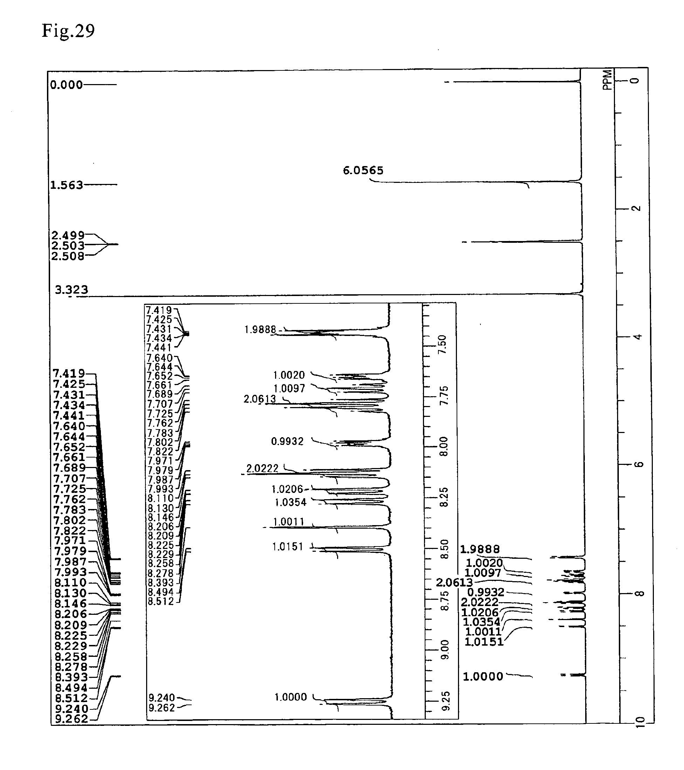

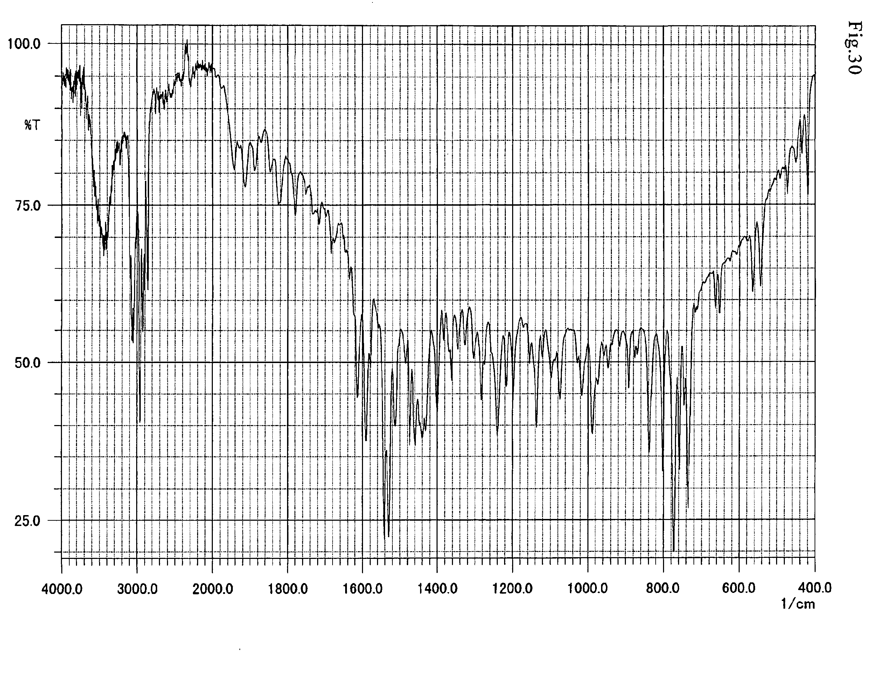

The identification of the target compound was made base on the NMR and IR spectrum charts of the target compound, which are respectively shown in Figure 29 and Figure 30.

### <Evaluation of Luminescent Properties>

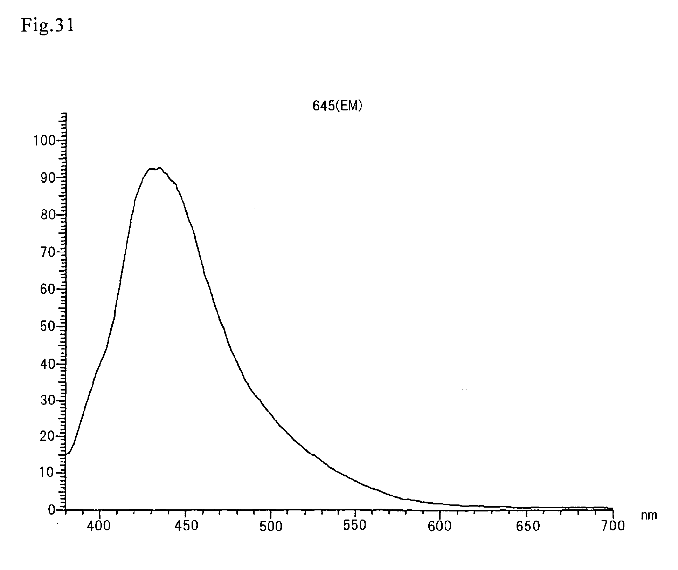

(1) An ITO substrate (dimensions: 50 mm x 50 mm; produced by Sanyo Vacuum Industries Co. , Ltd.) was ultrasonically cleaned in acetone for 10 minutes, then in 2-propanol for 10 minutes, and dried with nitrogen gas. In addition, the substrate was cleaned through the irradiation of UV rays for 5 minutes with a photo face processor/photo surface processor (produced by SEN Lights Corporation, wavelength: 254 nm).
   The cleaned ITO substrate was set in a vacuum metallizer (produced by DIAVAC Limited, model: UDS-M2-46) . α-NPD, the blue light-emitting compound of formula (35) produced in this example, and an aluminum alloy (Al:Li=99:1 (weight ratio), produced by Kojundo Chemical Laboratory Co., Ltd.) were deposited on the substrate in this order under 4 x 10⁻⁶ torr, so that an α-NPD layer with a thickness of 50 nm, a light-emitting layer with a thickness of 30 nm, and an electrode of the aluminum alloy with a thickness of 150 nm were formed on the substrate. Thus, a first luminescent element emitting blue light having a layered structure was prepared.
   The luminance and the chromaticity of the first luminescent element were measured with a BM-7 Fast measuring apparatus produced by TOPCON Corporation, with the voltage being raised gradually. The results were that, when the voltage was 14 V and the current was 18.47 mA, the luminance was 2,711.00 Cd/m², chromaticity X 0.2071, and chromaticity Y 0.3370.
(2) On an ITO substrate, which had been cleaned by ultrasonic treatment and UV rays irradiation, was formed a first film of PEDT, which is polyethylene dioxythiophene/polystyrene sulfonate produced by Bayer Corporation, by spin coating at 1000 rpm for 300 seconds. The first film was dried for 10 minutes at 200°C. Then, 70 mg of polyvinylcarbazole and 30 mg of the blue light-emitting compound represented by formula (35) were weighed out and dissolved in 5 ml of dichloroethane homogeneously. A second film of the obtained solution was formed on the first film of PEDT by spin coating at 1500 rpm for 3 seconds. On the second film was deposited an aluminum alloy (Al:Li=99:1 (weight ratio), produced by Kojundo Chemical Laboratory Co. , Ltd.) under 4 x 10⁻⁶ torr, so that an electrode of the aluminum alloy with a thickness of 150 Å was formed on the second film. Thus, a second luminescent element was prepared.
   The luminance and the chromaticity of the second luminescent element were measured with a BM-7 Fast measuring apparatus produced by TOPCON Corporation, with the voltage being raised gradually. The results were that, when the voltage was 21 V, the luminance was 105.6 Cd/m², chromaticity X 0.2328, and chromaticity Y 0.3059.
(3) A fluorescence spectrum of the blue light-emitting compound represented by formula (35) was measured with a model F-45000 spectrofluorophotometer (Exciting wavelength: 365 nm, Solvent: dioxane, Concentration: 0.25% by weight). The wavelength of the maximum emission was 435 nm. The measured spectrum is shown in Figure 31.

### (Example 12)

### -Synthesis of Blue Light-emitting Compound Represented by Formula (40)-

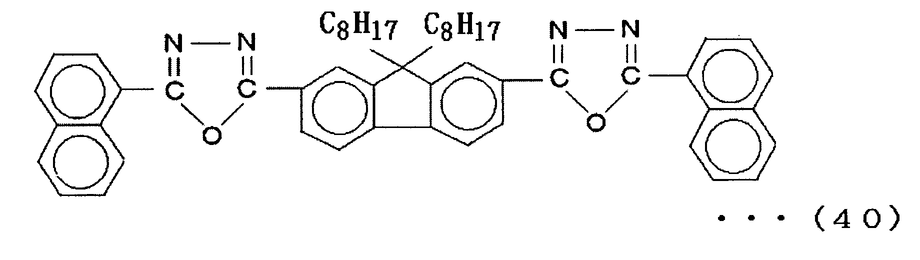

### <Synthesis of a Hydrazide>

The hydrazide represented by formula (19) was prepared by the same method as in Example 1.

### <Synthesis of an Acid Chloride>

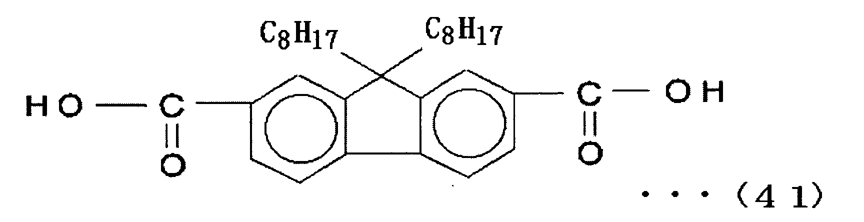

In a 1L pear-shaped flask were placed 9 g of the dicarboxylic acid represented by formula (41) above, 450 ml of dioxane, 3 g of pyridine, and 150 ml of thionyl chloride. The mixture in the flask was heated to 110°C in a silicone oil bath and kept at around the temperature for 2.5 hours. After the termination of the reaction, the solvent was distilled away with an evaporator. Solids were obtained. A column, which had been filled with silica gel, was charged with the solids, and the solids were purified with chloroform as a developer. 9.15 g of a light reddish white solidmatter, which was an acid chloride, was obtained.

### <Synthesis of an Intermediate for the Blue Light-emitting Compound>

In a 1L three-necked flask were placed 0.33 g of the light yellow solid matter (the hydrazide), 0.36 g of the reddish white solid matter (the acid chloride), 0.1 g of pyridine, and 200 ml of tetrahydrofuran. The reaction mixture in the flask was heated to 70°C in a silicone oil bath, and allowed to react for 15 hours at around the temperature. After the termination of the reaction, solids in the reaction product were separated and washed with water. Then the washed solids were dried up. 0.6 g of an intermediate for the blue light-emitting compound was obtained.

### <Synthesis of the Blue Light-emitting Compound>

In a 300 ml pear-shaped flask were placed 0.6 g of the intermediate, 25 ml of phosphoryl chloride, and 50 ml of dioxane. The solution in the pear-shaped flask was heated to 110°C in a silicone oil bath, and allowed to react for 13 hours at around the temperature. After the termination of the reaction, the reaction product was introduced into ice water. The resultant was subj ected to filtration by suction, and solids were collected. The collected solids were washed with water and methanol. Then washed solids were vacuum dried with a vacuum pump. 0.1 g of white crystals was obtained.

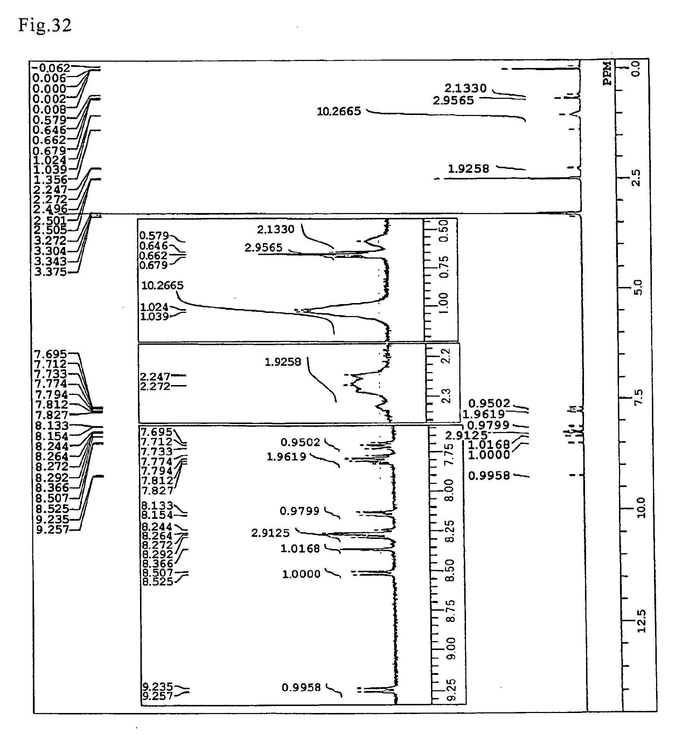

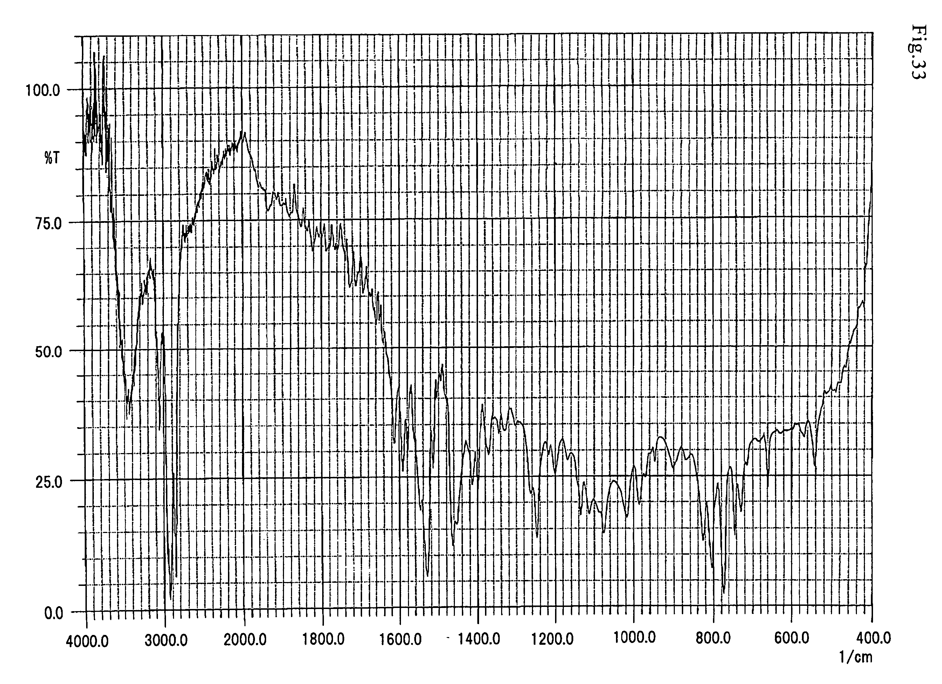

An NMR spectrum chart and an IR spectrum chart of the obtained crystals are respectively shown in Figure 32 and Figure 33. These data confirmed that the gel produced in this example were the blue light-emitting compound represented by formula (40).

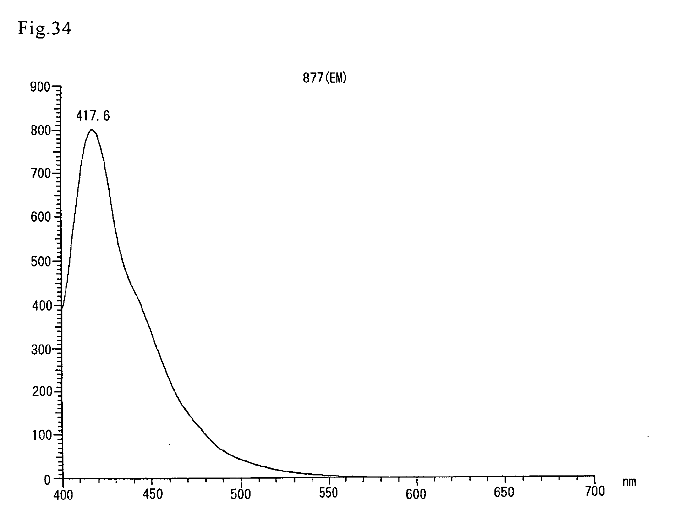

A fluorescence spectrum of the blue light-emitting compound prepared in this example was measured with a model F-45000 spectrofluorophotometer (Exciting wavelength: 365 nm, Solvent: DMAC, Concentration: 0.25% by weight). The wavelength of the maximum emission was 417.6 nm. The measured spectrum is shown in Figure 34.

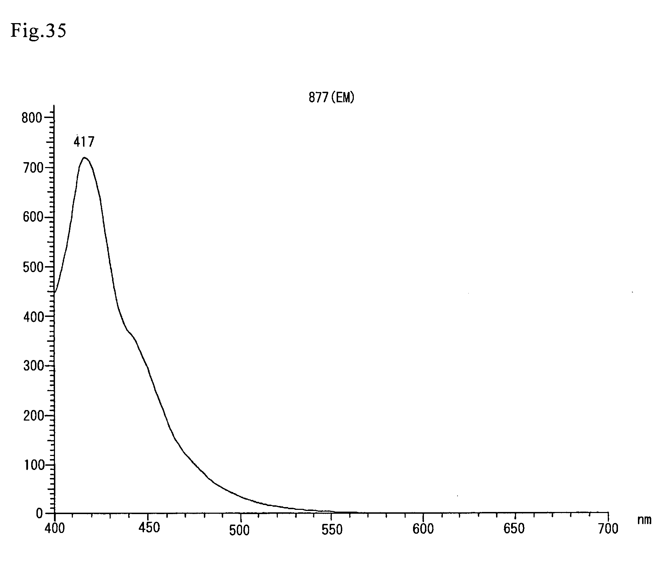

Another fluorescence spectrum of the blue light-emitting compound prepared in this example was measured with a model F-45000 spectrofluorophotometer (Exciting wavelength: 365 nm, Solvent: toluene, Concentration: 0.25% by weight). The wavelength of the maximum emission was 417 nm. The measured spectrum is shown in Figure 35.

### <Duration of Luminescence>

A solution was prepared by dissolving 5 mg of the blue light-emitting compound produced in Example 1 in 2 g of DMAC. The solution was irradiated with ultraviolet rays, and the intensity of light emitted by the solution was evaluated by three grades: strong, a little weak, and no luminance. As a result, blue light was emitted strongly for 400 days.

The blue light-emitting compound produced in this example, or Example 12, had solubility in a solvent, such as DMAC, six times larger than that produced in Example 1. Also, it is known that the duration of luminescence is proportional to the solubility of the compound in a solvent. Therefore, the blue light-emitting compound produced in Example 12 should have luminescence duration of about 5 to 6 years.

### Industrial Applicability

The present invention provides blue-light emitting compounds capable of ensuring the emission of blue light at a high luminance for a long time, processes of producing the compounds, and luminescent elements including the blue light-emitting compounds.

## Claims

1. A blue light-emitting compound having a chemical structure represented by formula (1): 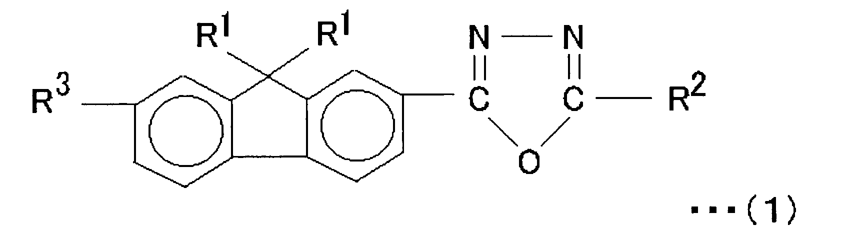 wherein R¹ is a hydrogen atom, an alkyl group having 1 to 15 carbon atoms, a cycloalkyl group having 6 to 15 carbon atoms, or an aryl group represented by one of formulas (1-1) to (1-4), wherein two R¹s may be the same or different from each other; R² is an aryl group represented one of formulas (1-1) to (1-4), or furyl group; and R³ is a group represented by formula (2) or a hydrogen atom;
the formula (1-1) is: 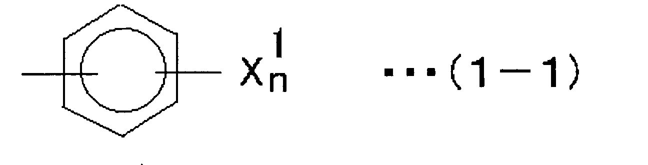 wherein X¹ is an alkyl group having 1 to 10 carbon atoms, an alkyl group having 1 to 10 carbon atoms, at least one hydrogen atom of which is replaced with a fluorine atom, or a hydrogen atom, and n denotes an integer of 1 to 5;
the formula (1-2) is: 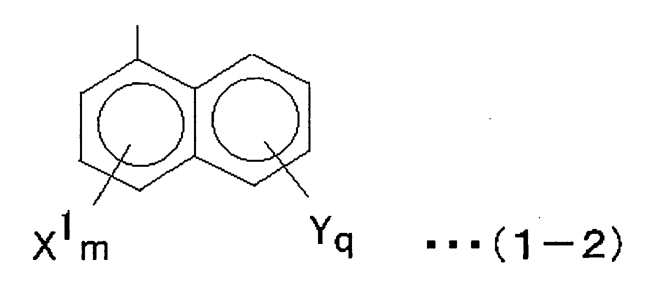 wherein X¹ means the same as the above; Y means an alkyl group having 1 to 10 carbon atoms, an alkyl group having 1 to 10 carbon atoms, at least one hydrogen atom of which is replaced with a fluorine atom, or a hydrogen atom; m denotes an integer from 1 to 3; q denotes an integer from 1 to 4; and X¹ and Y may be the same or different from each other;
the formula (1-3) is: 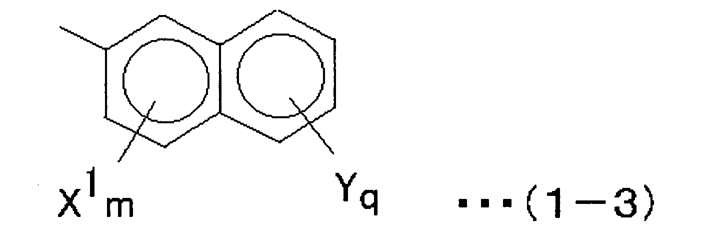 wherein X¹, Y, m and q denote the same as the above-defined, and X¹ and Y may be the same or different from each other;
the formula (1-4) is: 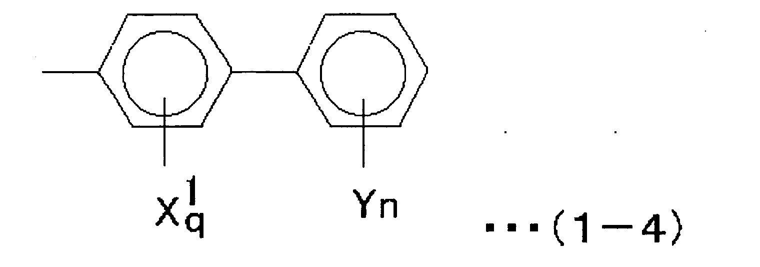 wherein X¹, Y, n, and q denote the same as those defined above, and X¹ and Y may be the same or different from each other; and
the formula (2) is: 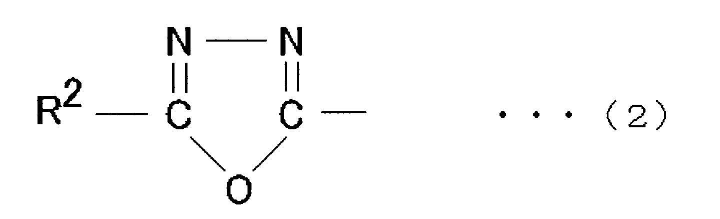 wherein R² denotes the same as that defined above; and when R³ in the formula (1) is the group represented by the formula (2), R² bonded to the oxadiazolyl group in the formula (2) may be the same as, or different from R² bonded to the oxadiazolyl group in the formula (1).

2. A blue light-emitting compound having a chemical structure represented by formula (3): 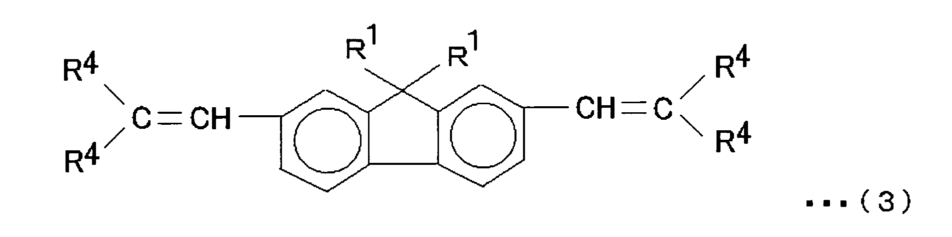 wherein R¹ denotes the same as that defined in claim 1; R⁴ denotes a hydrogen atom, or an aryl group represented by formula (3-1) or (3-2); and four R⁴s may be the same or different from each other;
the formula (3-1) is: 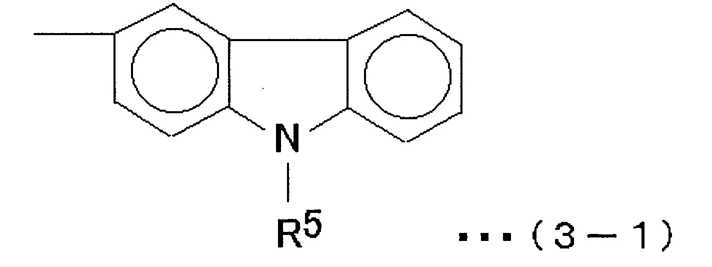 wherein R⁵ denotes a hydrogen atom or an alkyl group with 1 to 5 carbon atoms; and
the formula (3-2) is: 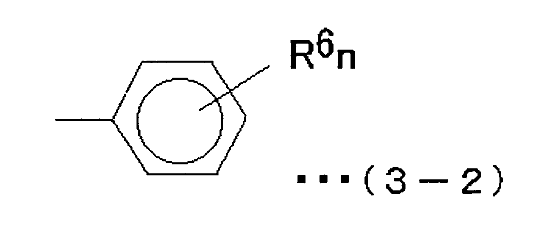 wherein R⁶ denotes a hydrogen atom or an alkyl group with 1 to 5 carbon atoms, and n denotes the same as that defined in claim 1.

3. A process for producing a blue light-emitting compound represented by formula (6), comprising reacting a dicarboxylic acid represented by formula (4) with a halogenating agent to produce a first acid chloride, reacting the first acid chloride with a hydrazide to produce a first intermediate for the blue light-emitting compound represented by formula (5), and dehydrating the first intermediate to produce the blue light-emitting compound represented by formula (6), wherein
the formula (4) is: 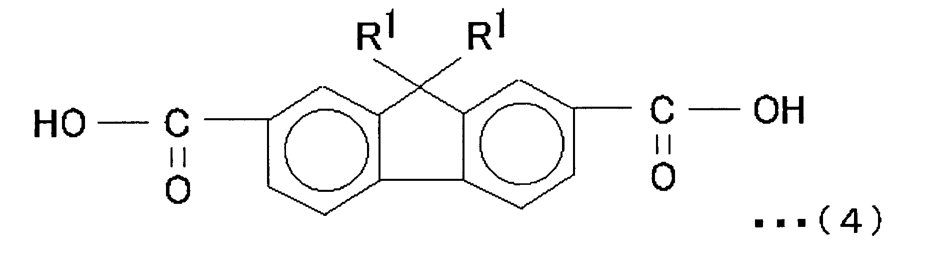 wherein R¹ denotes the same as that defined in claim 1;
the formula (5) is: 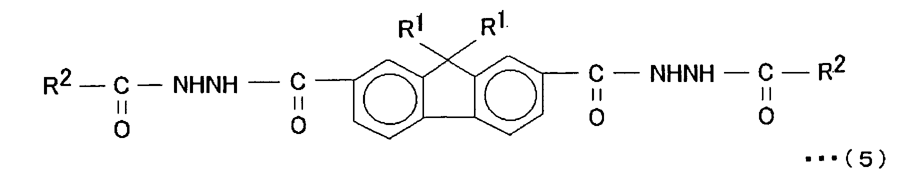 wherein R¹ and R² denote the same as those defined in claim 1; and
the formula (6) is: 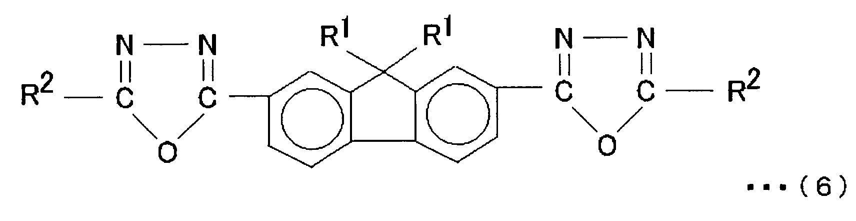 wherein R¹ and R² denote the same as those defined above.

4. A process for producing a blue light-emitting compound represented by formula (9), comprising reacting a carboxylic acid represented by formula (7) with a halogenating agent to produce a second acid chloride, reacting the second acid chloride with a hydrazide to produce a second intermediate represented by formula (8), and dehydrating the second intermediate to produce the blue light-emitting compound represented by formula (9), wherein
the formula (7) is: 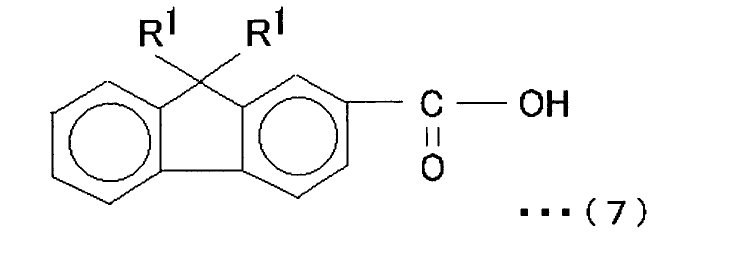 wherein R¹ is the same as that defined in claim 1;
the formula (8) is: 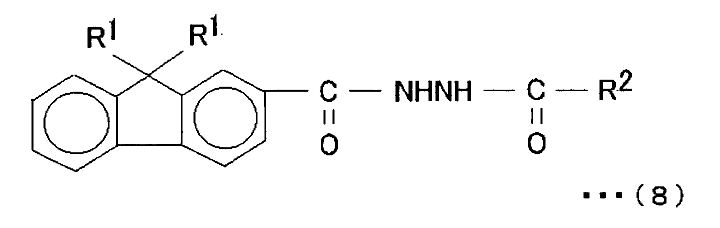 wherein R¹ and R² are the same as those defined in claim 1; and
the formula (9) is: 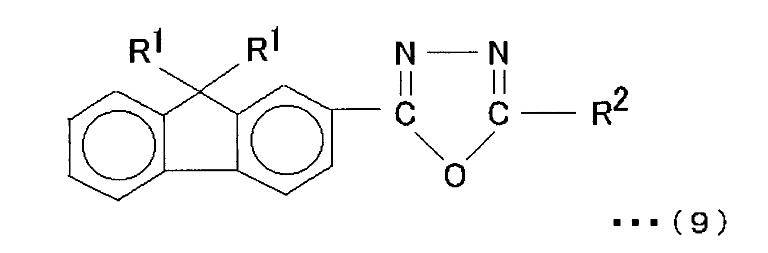 wherein R¹ and R² are the same as those defined above.

5. A process for producing the blue light-emitting compound represented by the formula (3) in claim 2, comprising halogenating a fluorene represented by formula (10) to produce an halogenated aromatic compound represented by formula (11), reacting the halogenated aromatic compound with triphenylphosphine to produce an organic phosphoric compound, and reacting the organic phosphoric compound with a carbonyl compound, wherein
the formula (10) is: 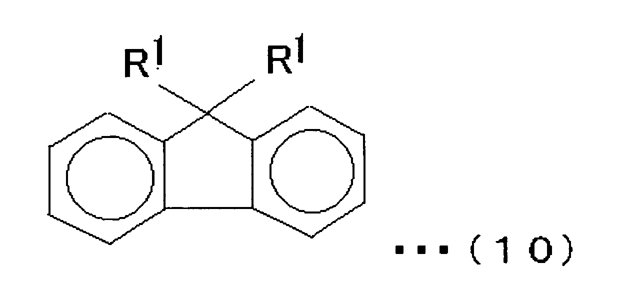 wherein R¹ denotes the same as that defined in claim 1; and
the formula (11) is: 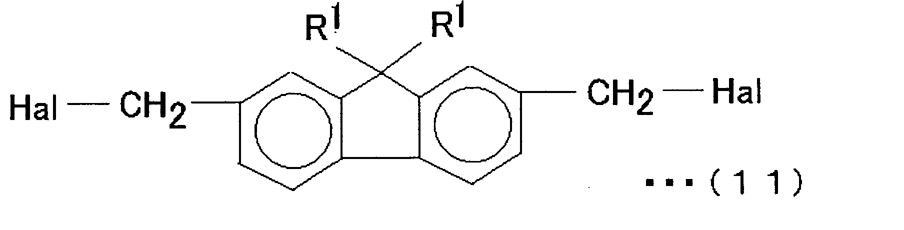 wherein R¹ denotes the same as that defined in claim 1, and "Hal" denotes a halogen atom.

6. A luminescent element comprising a light-emitting layer including the blue light-emitting compound represented by the formula (1) or (3) between a pair of electrodes.

## Amended claims

### Amended claims under Art. 19.1 PCT

**1.** deleted.

**2.** (amended) A blue light-emitting compound having a chemical structure represented by formula (3): 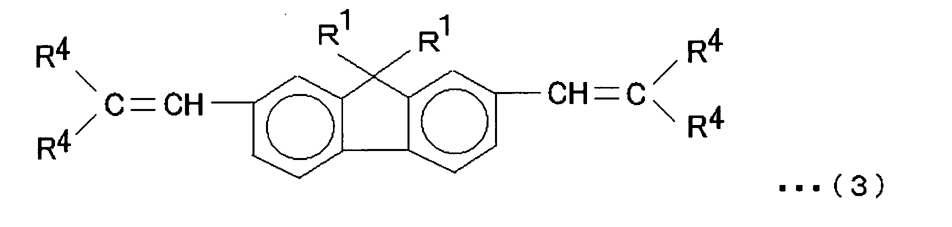 wherein R¹ is a hydrogen atom, an alkyl group having 1 to 15 carbon atoms, a cycloalkyl group having 6 to 15 carbon atoms, or an aryl group represented by one of formulas (1-1) to (1-4), wherein two R¹s may be the same or different from each other; and R⁴ denotes a hydrogen atom, an aryl group represented by formula (3-1), or phenyl group, wherein four R⁴s may be the same or different from each other;
the formula (1-1) is: 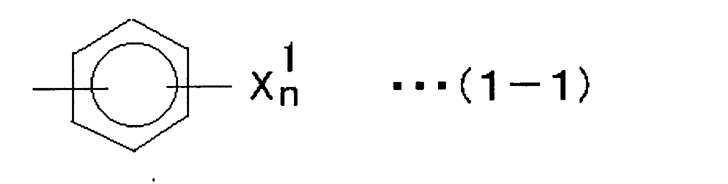 wherein X¹ is an alkyl group having 1 to 10 carbon atoms, an alkyl group having 1 to 10 carbon atoms, at least one hydrogen atom of which is replaced with a fluorine atom, or a hydrogen atom, and n denotes an integer of 1 to 5;
the formula (1-2) is: 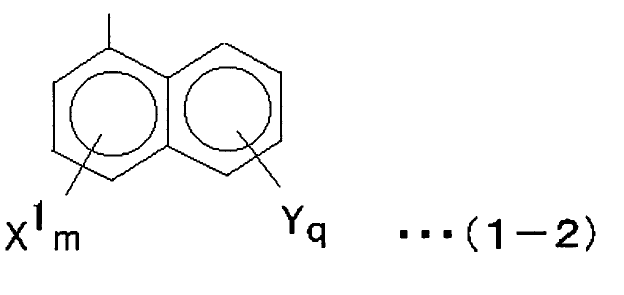 wherein X¹ means the same as the above; Y means an alkyl group having 1 to 10 carbon atoms, an alkyl group having 1 to 10 carbon atoms, at least one hydrogen atom of which is replaced with a fluorine atom, or a hydrogen atom; m denotes an integer from 1 to 3; q denotes an integer from 1 to 4; and X¹ and Y may be the same or different from each other;
the formula (1-3) is: 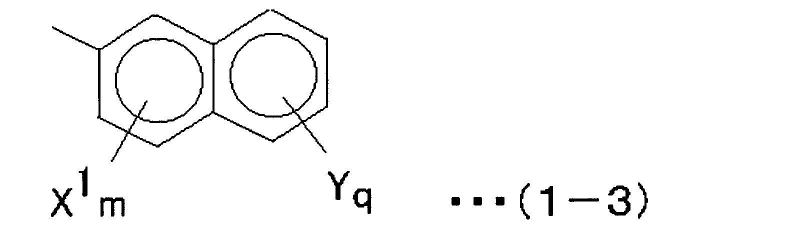 wherein X¹, Y, m and q denote the same as the above-defined, and X¹ and Y may be the same or different from each other;
the formula (1-4) is: 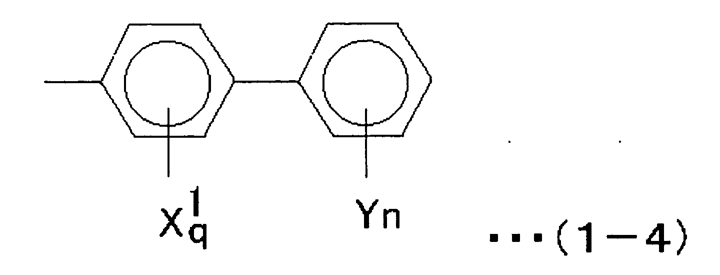 wherein X¹, Y, n, and q denote the same as those defined above, and X¹ and Y may be the same or different from each other; and
the formula (3-1) is: 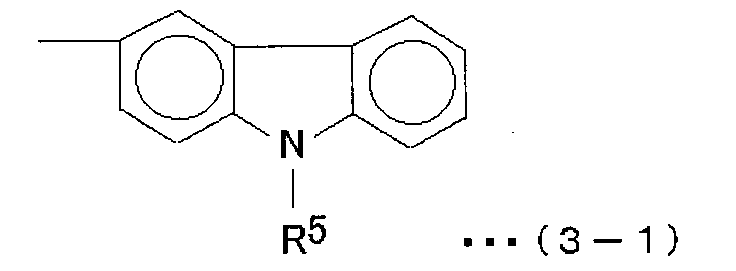 wherein R⁵ denotes a hydrogen atom or an alkyl group with 1 to 5 carbon atoms.

**3.** deleted.

**4.** deleted.

**5.** amended) A process for producing the blue light-emitting compound represented by the formula (3) in claim 2, comprising halogenating a fluorene represented by formula (10) to produce an halogenated aromatic compound represented by formula (11), reacting the halogenated aromatic compound with triphenylphosphine to produce an organic phosphoric compound, and reacting the organic phosphoric compound with a carbonyl compound, wherein
the formula (10) is: 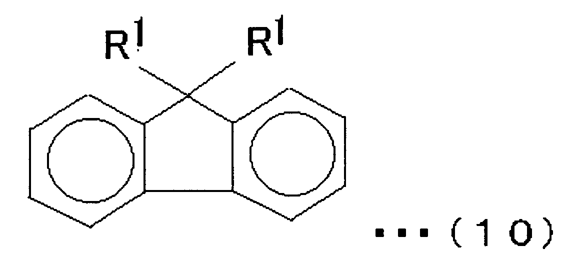 wherein R¹ denotes the same as that defined in claim 2; and
the formula (11) is: 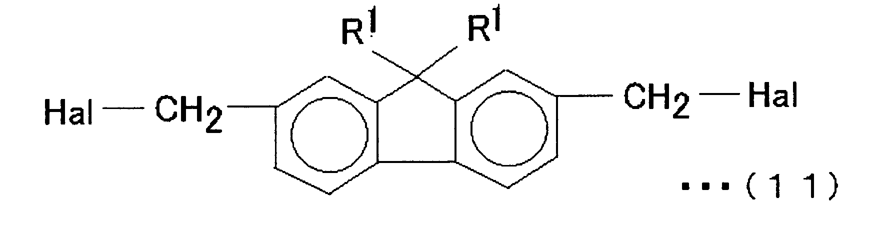 wherein R¹ denotes the same as that defined in claim 2, and "Hal" denotes a halogen atom.

**6.** (amended) A luminescent element comprising a light-emitting layer including the blue light-emitting compound represented by the formula (3) between a pair of electrodes

Statement under Art. 19.1 PCT

Claim 1 is deleted from the application.
Claim 2 is amended as shown above.

Reference 3 (JP2003-64003A) teaches a compound represented by formula (1). In the compound, a benzene ring that has a substituent is bonded to each of the unsaturated groups having a double bond that are bonded to the fluorene skeleton. On the other hand, the compound represented by formula (3) in claim 2 takes embodiments different from the disclosure of Reference 3. Examples of the embodiments are as follows: (A) one benzene ring that does not have any substituents is bonded to each of the vinyl groups bonded to the fluorene skeleton, (B) two aromatic groups are bonded to each unsaturated group having a double bond that is bonded to the fluorene skeleton, and (C) one carbazole skeleton is bonded to each unsaturated group having a double bond that is bonded to the fluorene skeleton. The compound recited in amended claim 2 is not taught in Reference 3. Also, Reference 3 neither discloses nor suggests that the benzene ring having a substituent, which is bonded with the unsaturated group having a double bond that in turn is bonded to the fluorene skeleton, may be replaced with other substituents.
